# EUROPEAN PATENT SPECIFICATION

(11) **EP 4 345 444 B1**
(45) Date of publication and mention of the grant of the patent: **06.05.2026**
(21) Application number: 23200665.0
(22) Date of filing: 28.09.2023
(51) Int. Cl.: G01N 21/27, C12Q 1/6825, C12Q 1/6869, G01N 21/64, G01N 21/05

(54) **DYNAMIC OPTICAL SYSTEM CALIBRATION**
DYNAMISCHE KALIBRIERUNG EINES OPTISCHEN SYSTEMS
ÉTALONNAGE DE SYSTÈME OPTIQUE DYNAMIQUE

(30) Priority: 29.09.2022 US 202263411300 P; 15.06.2023 US 202363521136 P
(43) Date of publication of application: 03.04.2024
(60) Divisional application: 26166516.0
(73) Proprietor: Illumina, Inc., San Diego, CA 92122 (US)
(72) Inventor: BLAIR, Dustin, San Diego, 92122 (US); WEN, Patrick, San Diego, 92122 (US); EARNEY, John, San Diego, 92122 (US); PRABHU, Anmiv, San Diego, 92122 (US); ABASKHARON, Rachel, San Diego, 92122 (US); HOLST, Gregory, San Diego, 92122 (US); LIU, Chia-Hsi, San Diego, 92122 (US); THAKUR, Ravi Bhushan Singhchawhan, San Diego, 92122 (US); WATSON, Dakota, San Diego, 92122 (US); BARTIG, Kevin, San Diego, 92122 (US); SIM, Daeyong, San Diego, 92122 (US)
(74) Representative: Murgitroyd & Company

(56) References cited:
- EP-A1- 3 373 400
- EP-A2- 3 988 985
- US-A1- 2010 111 768
- US-A1- 2010 157 086
- US-A1- 2010 208 961
- US-A1- 2018 258 468
- US-A1- 2022 150 394

## Description

### BACKGROUND

The subject matter discussed in this section should not be assumed to be prior art merely as a result of its mention in this section. Similarly, a problem mentioned in this section or associated with the subject matter provided as background should not be assumed to have been previously recognized in the prior art. The subject matter in this section merely represents different approaches, which in and of themselves may also correspond to implementations of the claimed technology.

Aspects of the present disclosure relate generally to biological or chemical analysis and more particularly to systems and methods using image sensors for biological or chemical analysis.

Various protocols in biological or chemical research involve performing a large number of controlled reactions on local support surfaces or within predefined reaction chambers. The designated reactions may then be observed or detected, and subsequent analysis may help identify or reveal properties of chemicals involved in the reaction. For example, in some multiplex assays, an unknown analyte having an identifiable label (e.g., fluorescent label) may be exposed to thousands of known probes under controlled conditions. Each known probe may be deposited into a corresponding well of a flow cell channel. Observing any chemical reactions that occur between the known probes and the unknown analyte within the wells may help identify or reveal properties of the analyte. Other examples of such protocols include known DNA sequencing processes, such as sequencing-by-synthesis (SBS) or cyclic-array sequencing.

In some conventional fluorescent-detection protocols, an optical system is used to direct an excitation light onto fluorescently-labeled analytes and to also detect the fluorescent signals that may be emitted from the analytes. Such optical systems may include an arrangement of lenses, filters, and light sources. It may be desirable to provide calibration of such optical systems without substantially affecting overall processing times.
US2010/111768 A1 describes systems and devices for sequencing of nucleic acid, such as short DNA sequences from clonally amplified single-molecule arrays. US2010/157086 A1 describes a method and system for controlling focus dynamically of a sample imager, the method comprising scanning a sample with an optical assembly that apportions the sample into regions based on a scan pattern. EP3988985 A2 describes methods and systems for automatically focusing multiple images of one or more objects on a substrate. US2010/208961 A1 describes a method for determining the quality of focus of a digital image of a biological specimen including obtaining a digital image of a specimen using a specimen imaging apparatus. A measure of image texture is calculated at two different scales, and the measurements are compared to determine how much high-resolution data the image contains compared to low-resolution data. US2022/150394 A1 describes a method used to generate an analysis image of a moving sample based on one or more exposures. US2018/258468 A1 describes a laser line illuminator for high throughput sequencing. Imaging systems including an objective lens and a line generation module are described. The objective lens may focus a first light beam emitted by the line generation module and a second light beam emitted by the line generation module at a focal point external to a sample so as to adjust line width. EP3373400 A1 describe systems and methods for improved focus tracking using a hybrid mode light source, the systems and methods including an imaging system that may include a laser diode source; an objective lens positioned to direct a focus tracking beam from the light source onto a location in a sample container and to receive the focus tracking beam reflected from the sample; and an image sensor that may include a plurality of pixel locations to receive focus tracking beam that is reflected off of the location in the sample container, where the reflected focus tracking beam may create a spot on the image sensor.

### BRIEF DESCRIPTION OF THE DRAWINGS

FIG. 1 depicts a schematic diagram of an example of an imaging assembly that may be implemented in a system for biological or chemical analysis.
FIG. 2 depicts a perspective view of an example of a flow cell that may be utilized with the system of FIG. 1.
FIG. 3 depicts an enlarged perspective view of a channel of the flow cell of FIG. 3.
FIG. 4 depicts a top plan view of another example of a flow cell that may be utilized with the system of FIG. 1
FIG. 5 depicts an enlarged top plan view of a channel of the flow cell of FIG. 4.
FIG. 6 depicts a graph depicting an example of image capture positions during a focus model generation process.
FIG. 7 depicts a motion profile depicting an example of an integrated through focus path for moving an objective lens of an imaging assembly for focus model generation and/or updating.
FIG. 8 depicts a graph detecting focus tracking spots reflected by a first surface and a second surface.
FIG. 9 depicts a pair of graphs showing a substantially linear relationship between the position of a detected focus tracking spot relative to a z position height of an objective of an imaging assembly.
FIG. 10 depicts a graph showing average spot separation values through a calibration region of a flow cell.
FIG. 11 depicts a graph showing image quality score values through a calibration region of a flow cell.
FIG. 12 depicts a graph showing image quality score values relative to average spot separation values.
FIG. 13 depicts a flow chart representing an example of a method of dynamically calibrating optical system components.
FIG. 14 depicts a flow chart representing an example of a method of dynamically calibrating optical system components.
FIG. 15 depicts an exemplary set of regions of interest.
FIG. 16 depicts a particular approach which may be taken when performing calibration acts.

### DETAILED DESCRIPTION

According to a first aspect of the present invention, there is provided an apparatus as claimed in claim 1. According to a second aspect of the invention, there is provided a method as claimed in claim 9.

### I. Overview of System for Biological or Chemical Analysis

Described herein are devices, systems, and methods for dynamically optically calibrating an imaging assembly for a biological or chemical analysis system. Dynamic optical calibration may improve performance of the biological or chemical analysis system by improving image quality at one or more points of a substrate of interest during a scanning process. Examples described herein may be used in various biological or chemical processes and systems for academic analysis, commercial analysis, or other analysis. More specifically, examples described herein may be used in various processes and systems where it is desired to detect an event, property, quality, or characteristic that is indicative of a designated reaction.

Bioassay systems such as those described herein may be configured to perform a plurality of designated reactions that may be detected individually or collectively. The biosensors and bioassay systems may be configured to perform numerous cycles in which a plurality of designated reactions occurs in parallel. For example, the bioassay systems may be used to sequence a dense array of nucleic acid features through iterative cycles of enzymatic manipulation and image acquisition. Cartridges and biosensors that are used in the bioassay systems may include one or more microfluidic channels that deliver reagents or other reaction components to a reaction site. The reaction sites may be randomly distributed across a substantially planar surface; or may be patterned across a substantially planar surface in a predetermined manner, such as in a hexagonal pattern, a rectilinear pattern, or any other repeating pattern. In some versions, the reaction sites are located in reaction chambers that compartmentalize the designated reactions therein.

Regardless of the form taken by the reaction sites, each of the reaction sites may be imaged to detect light from the reaction site. In some examples, one or more image sensors may detect light emitted from reaction sites. The signals indicating photons emitted from the reaction sites and detected by the individual image sensors may be referred to as those sensors' illumination values. These illumination values may be combined into an image indicating photons as detected from the reaction sites. These images may be further analyzed to identify compositions, reactions, conditions, etc., at each reaction site.

The following detailed description of certain examples will be better understood when read in conjunction with the appended drawings. To the extent that the figures illustrate diagrams of the functional blocks of various examples, the functional blocks are not necessarily indicative of the division between hardware components. Thus, for example, one or more of the functional blocks (e.g., processors or memories) may be implemented in a single piece of hardware (e.g., a general purpose signal processor or random access memory, hard disk, or the like). Similarly, the programs may be standalone programs, may be incorporated as subroutines in an operating system, may be functions in an installed software package, and the like. It should be understood that the various examples are not limited to the arrangements and instrumentality shown in the drawings.

As used herein, an element or step recited in the singular and proceeded with the word "a" or "an" should be understood as not excluding plural of said elements or steps, unless such exclusion is explicitly stated. Furthermore, references to "one example" are not intended to be interpreted as excluding the existence of additional examples that also incorporate the recited features. Moreover, unless explicitly stated to the contrary, examples "comprising" or "having" an element or a plurality of elements having a particular property may include additional elements whether or not they have that property.

As used herein, a "designated reaction" includes a change in at least one of a chemical, electrical, physical, or optical property (or quality) of an analyte-of-interest. In some examples, the designated reaction is a positive binding event (e.g., incorporation of one or more fluorescently labeled biomolecules with the analyte-of-interest). More generally, the designated reaction may be a chemical transformation, chemical change, or chemical interaction. In some examples, the designated reaction includes the incorporation of a fluorescently-labeled molecule to an analyte. The analyte may be an oligonucleotide and the fluorescently-labeled molecule may be a nucleotide. The designated reaction may be detected when an excitation light is directed toward the oligonucleotide having the labeled nucleotide, and the fluorophore of the labeled nucleotide emits a detectable fluorescent signal. In alternative examples, the detected fluorescence is a result of chemiluminescence or bioluminescence. A designated reaction may also increase fluorescence (or Förster) resonance energy transfer (FRET), for example, by bringing a donor fluorophore in proximity to an acceptor fluorophore, decrease FRET by separating donor and acceptor fluorophores, increase fluorescence by separating a quencher from a fluorophore or decrease fluorescence by co-locating a quencher and fluorophore.

As used herein, a "reaction component" or "reactant" includes any substance that may be used to obtain a designated reaction. For example, reaction components include reagents, enzymes, samples, other biomolecules, and buffer solutions. The reaction components may be delivered to a reaction site in a solution and/or immobilized at a reaction site. The reaction components may interact directly or indirectly with another substance, such as the analyte-of-interest.

As used herein, the term "reaction site" is a localized region where a designated reaction may occur. A reaction site may include support surfaces of a substrate where a substance may be immobilized or aligned thereon. For example, a reaction site may include a substantially planar surface in a channel of a flow cell that has a colony of nucleic acids thereon. The nucleic acids in the colony may have substantially the same sequence, being for example, clonal copies of a single stranded or double stranded template. In some instances, the nucleic acids in the colony may be polyclonal by having two or more populations of clonal copies of different templates. In some implementations, the polyclonal colonies may still be detectible if one of the clonal copies is distinguishable over the other clonal copies. However, in some examples a reaction site may contain only a single nucleic acid molecule, for example, in a single stranded or double stranded form. Furthermore, a plurality of reaction sites may be randomly distributed along the support surface or arranged in a predetermined manner (e.g., side-by-side in a matrix, such as in microarrays). A reaction site may also include a reaction chamber that at least partially defines a spatial region or volume configured to compartmentalize the designated reaction. As used herein, the term "reaction chamber" includes a spatial region that is in fluid communication with a flow channel. The reaction chamber may be at least partially separated from the surrounding environment or other spatial regions. For example, a plurality of reaction chambers may be separated from each other by shared walls, by a height difference of bottom surfaces, by vertical locations along a sidewall, or other distinguishable separation features. As a more specific example, the reaction chamber may include a cavity defined by interior surfaces of a well and have an opening or aperture so that the cavity may be in fluid communication with a flow channel. Reaction sites do not necessarily need to be provided in reaction chambers and may instead be provided on or in any other suitable kind of structure.

As used herein, the term "adjacent" when used with respect to two reaction sites means no other reaction site is located between the two reaction sites. The term "adjacent" may have a similar meaning when used with respect to adjacent detection paths and adjacent image sensors (e.g., adjacent image sensors have no other image sensor therebetween). In some cases, a reaction site may not be adjacent to another reaction site; but may still be within an immediate vicinity of the other reaction site. A first reaction site may be in the immediate vicinity of a second reaction site when fluorescent emission signals from the first reaction site are detected by the image sensor associated with the second reaction site. More specifically, a first reaction site may be in the immediate vicinity of a second reaction site when the image sensor associated with the second reaction site detects, for example, crosstalk from the first reaction site. Adjacent reaction sites may be contiguous such that they abut each other or the adjacent sites may be non-contiguous having an intervening space between, such as interstitial spaces.

As used herein, a "substance" includes items or solids, such as capture beads, as well as biological or chemical substances. As used herein, a "biological or chemical substance" includes biomolecules, samples-of-interest, analytes-of-interest, and other chemical compound(s). A biological or chemical substance may be used to detect, identify, or analyze other chemical compound(s), or function as intermediaries to study or analyze other chemical compound(s). In particular examples, the biological or chemical substances include a biomolecule. As used herein, a "biomolecule" includes at least one of a biopolymer, nucleoside, nucleic acid, polynucleotide, oligonucleotide, protein, enzyme, polypeptide, antibody, antigen, ligand, receptor, polysaccharide, carbohydrate, polyphosphate, cell, tissue, organism, or fragment thereof or any other biologically active chemical compound(s) such as analogs or mimetics of the aforementioned species.

Biomolecules, samples, and biological or chemical substances may be naturally occurring or synthetic and may be suspended in a solution or mixture within a spatial region. Biomolecules, samples, and biological or chemical substances may also be bound to a solid phase or gel material. Biomolecules, samples, and biological or chemical substances may also include a pharmaceutical composition. In some cases, biomolecules, samples, and biological or chemical substances of interest may be referred to as targets, probes, or analytes.

As used herein, when the terms "removably" and "coupled" (or "engaged") are used together to describe a relationship between components, the term is intended to mean that a connection between the components is readily separable without destroying or damaging the components. Components are readily separable when the components may be separated from each other without undue effort, or without a significant amount of time spent, in separating the components. For example, components may be removably coupled or engaged in an electrical manner such that the mating contacts of the components are not destroyed or damaged. Components may also be removably coupled or engaged in a mechanical manner such that the features that hold a component are not destroyed or damaged. Components may also be removably coupled or engaged in a fluidic manner such that ports of a component are not destroyed or damaged. The component is not considered to be destroyed or damaged if, for example, only a simple adjustment to the component (e.g., realignment) or a simple replacement (e.g., replacing a nozzle) is required.

As used herein, the term "fluid communication" or "fluidically coupled" refers to two spatial regions being connected together such that a liquid or gas may flow between the two spatial regions. For example, a microfluidic channel may be in fluid communication with a reaction chamber such that a fluid may flow freely into the reaction chamber from the microfluidic channel. The terms "in fluid communication" or "fluidically coupled" allow for two spatial regions being in fluid communication through one or more valves, restrictors, or other fluidic components to control or regulate a flow of fluid through a system.

In some examples, nucleic acids can be attached to a surface and amplified. Examples of such amplification are described in U.S. Pat. No. 7,741,463, entitled "Method of Preparing Libraries of Template Polynucleotides," issued June 22, 2010; and/or U.S. Pat. No. 7,270,981, entitled "Recombinase Polymerase Amplification," issued September 18, 2007. In some cases, repeated rounds of extension (e.g., amplification) using an immobilized primer and primer in solution may provide multiple copies of the nucleic acid.

In particular examples, the assay protocols executed by the systems and methods described herein include the use of natural nucleotides and also enzymes that are configured to interact with the natural nucleotides. Natural nucleotides include, for example, ribonucleotides or deoxyribonucleotides. Natural nucleotides can be in the mono-, di-, or tri-phosphate form and can have a base selected from adenine (A), Thymine (T), uracil (U), guanine (G) or cytosine (C). It will be understood however that non-natural nucleotides, modified nucleotides, or analogs of the aforementioned nucleotides can be used.

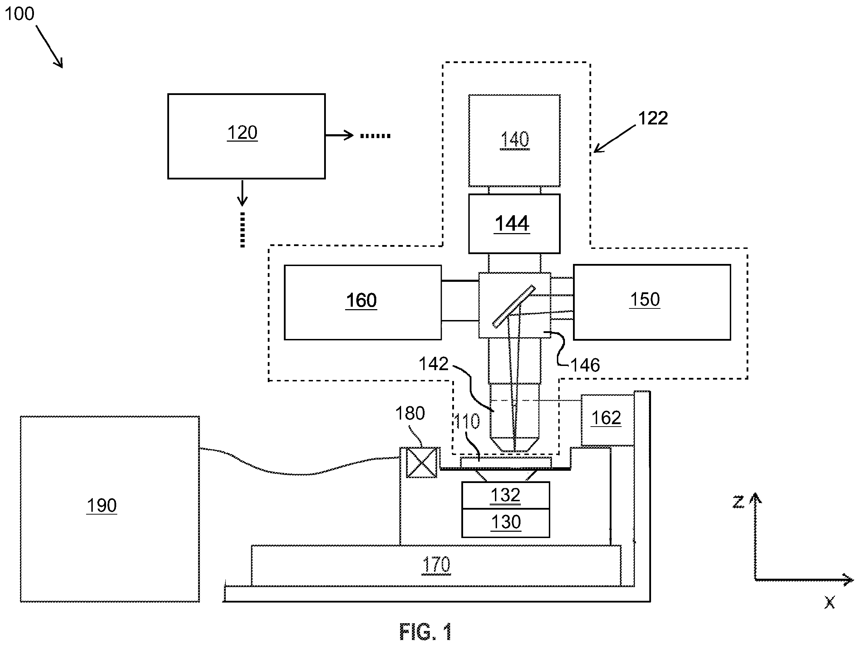

FIG. 1 depicts an example of components of a system (100) that may be used to provide biological or chemical analysis. In some examples, system (100) is a workstation that may be similar to a bench-top device. For example, a majority (or all) of the systems and components for conducting the designated reactions may be within a common housing. In particular examples, system (100) is a nucleic acid sequencing system (or sequencer) configured for various applications, including but not limited to de novo sequencing, resequencing of whole genomes or target genomic regions, and metagenomics. The sequencer may also be used for DNA or RNA analysis. In some versions, system (100) may also be configured to generate reaction sites in a flow cell (110). For example, system (100) may be configured to receive a sample and generate surface attached clusters of clonally or substantially clonal amplified nucleic acids derived from the sample. In some implementations, the cluster may comprise a particular sample that is a distinguishable portion of the cluster even if the cluster is polyclonal as a result of one or more other samples being present within the cluster. System (100) is further configured to utilize an imaging assembly (122) to capture images of the reaction sites on flow cell (110).

In particular examples, the system (100) is to perform a large number of parallel reactions within flow cell (110). Flow cell (110) includes one or more reaction sites where designated reactions may occur. The reaction sites may be, for example, immobilized to or aligned on a solid surface of flow cell (110) or immobilized to beads (or other movable substrates) that are located within corresponding reaction chambers of flow cell (110). The reaction sites may include, for example, clusters of clonally amplified nucleic acids. Flow cell (110) may include one or more flow channels that receive a solution from the system (100) and direct the solution toward the reaction sites. Optionally, flow cell (110) may engage a thermal element for transferring thermal energy into or out of the flow channel.

System (100) may include various components, assemblies, and systems (or sub-systems) that interact with each other to perform a predetermined method or assay protocol for biological or chemical analysis. For example, system (100) includes a system controller (120) that may communicate with the various components, assemblies, and sub-systems of the system (100). Examples of such components are described in greater detail below. Controller (120) may include one or more microprocessors, storage devices, and/or any other suitable electrical components that are configured to cooperate to execute control algorithms, data processing, etc.

In the present example, imaging assembly (122) includes a light emitting assembly (150) that emits light that reaches reaction sites on flow cell (110). Light emitting assembly (150) may include an incoherent light emitter (e.g., emit light beams output by one or more excitation diodes), or a coherent light emitter such as emitter of light output by one or more lasers or laser diodes. In some implementations, light emitting assembly (150) may include a plurality of different light sources (not shown), each light source emitting light of a different wavelength range. Some versions of light emitting assembly (150) may also include one or more collimating lenses (not shown), a light structuring optical assembly (not shown), a projection lens (not shown) that is operable to adjust a structured beam shape and path, epifluorescence microscopy components, and/or other components. Although system (100) is illustrated as having a single light emitting assembly (150), multiple light emitting assemblies (150) may be included in some other implementations.

In the present example, the light from light emitting assembly (150) is directed by dichroic mirror assembly (146) through an objective lens assembly (142) onto a sample of a flow cell (110), which is positioned on a motion stage (170). In the case of fluorescent microscopy of a sample, a fluorescent element associated with the sample of interest fluoresces in response to the excitation light, and the resultant light is collected by objective lens assembly (142) and is directed to an image sensor of camera system (140) to detect the emitted fluorescence. In some implementations, a tube lens assembly may be positioned between the objective lens assembly (142) and the dichroic mirror assembly (146) or between the dichroic mirror (146) and the image sensor of the camera system (140). A moveable lens element may be translatable along a longitudinal axis of the tube lens assembly to account for focusing on an upper interior surface or lower interior surface of the flow cell (110) and/or spherical aberration introduced by movement of the objective lens assembly (142).

In the present example, a filter switching assembly (144) is interposed between dichroic mirror assembly (146) and camera system (140). Filter switching assembly (144) includes one or more emission filters that may be used to pass through particular ranges of emission wavelengths and block (or reflect) other ranges of emission wavelengths. For example, the one or more emission filters may be used to direct different wavelength ranges of emitted light to different image sensors of the camera system (140) of imaging assembly (122). For instance, the emission filters may be implemented as dichroic mirrors that direct emission light of different wavelengths from flow cell (110) to different image sensors of camera system (140). In some variations, a projection lens is interposed between filter switching assembly (144) and camera system (140). Filter switching assembly (144) may be omitted in some versions.

In the example of system (100), fluid delivery module or device (190) may direct the flow of reagents (e.g., fluorescently labeled nucleotides, buffers, enzymes, cleavage reagents, etc.) to (and through) flow cell (110) and waste valve (180). Flow cell (110) may include one or more substrates upon which the samples are provided. For example, in the case of a system to analyze a large number of different nucleic acid sequences, flow cell (110) may include one or more substrates on which nucleic acids to be sequenced are bound, attached, or associated. The substrate may include any inert substrate or matrix to which nucleic acids may be attached, such as for example glass surfaces, plastic surfaces, latex, dextran, polystyrene surfaces, polypropylene surfaces, polyacrylamide gels, gold surfaces, and silicon wafers. In some applications, the substrate is within a channel or includes a channel formed within the substrate or other area at a plurality of locations formed in a matrix or array across the flow cell (110). System (100) may also include a temperature station actuator (130) and heater/cooler (132) that may optionally regulate the temperature of conditions of the fluids within the flow cell (110). In some implementations, the heater/cooler (132) may be fixed to a sample stage (170) upon which the flow cell (110) is placed and/or may be integrated into sample stage (170).

In some versions, the flow cell (110) may be implemented as a patterned flow cell including a transparent cover plate, a substrate, and configured to contain a liquid therebetween, and a biological sample may be located at an inside surface of the transparent cover plate and/or an inside surface of the substrate. The flow cell may include a large number (e.g., thousands, millions, or billions) of wells (also referred to as nanowells) or regions that are patterned into a defined array (e.g., a hexagonal array, rectangular array, etc.) into the substrate. Such wells may define reaction chambers providing reaction sites as described above. Each region may form a cluster (e.g., a monoclonal cluster, a substantially monoclonal cluster, or a polyclonal cluster) or more one than cluster of a biological sample such as DNA, RNA, or another genomic material that may be sequenced, for example, using sequencing by synthesis. A substantially monoclonal cluster may be one where a particular sample forms a distinguishable portion of the cluster even if the cluster itself is polyclonal as a result of one or more other samples being present within the cluster. The flow cell may be further divided into a number of spaced apart lanes (e.g., eight lanes), each lane including a hexagonal array of clusters or a rectilinear array of clusters.

Flow cell (110) may be mounted on sample stage (170), which may provide movement and alignment of flow cell (110) relative to objective lens assembly (142). Sample stage (170) may have one or more actuators to allow sample stage (170) to move in any of three dimensions. For example, in terms of the Cartesian coordinate system, actuators may be provided to allow sample stage (170) to move in the x, y, and z directions relative to objective lens assembly (142), tilt relative to objective lens assembly (142), and/or otherwise move relative to objective lens assembly (142). Movement of sample stage (170) may allow one or more sample locations on flow cell (110) to be positioned in optical alignment with objective lens assembly (142). Movement of sample stage (170) relative to objective lens assembly (142) may be achieved by moving sample stage (170) itself, by moving objective lens assembly (142), by moving some other component of imaging assembly (122), by moving some other component of system (100), or any combination of the foregoing. For instance, in some implementations, the sample stage (170) may be actuatable in the X and Y directions relative to the objective lens assembly (142) while a focus component (162) or Z-stage may move the objective lens assembly (142) along the Z direction relative to the sample stage (170). Further implementations may also include moving imaging assembly (122) over a stationary flow cell (110). Thus, in some versions, flow cell (110) may be fixed during imaging while one or more components of imaging assembly (122) is/are moved to capture images at different regions of flow cell (110).

In some implementations, a focus component (162) may be included to control positioning of the objective lens relative to the flow cell (110) in the focus direction (e.g., along the z-axis or z-dimension). Focus component (162) may include one or more actuators physically coupled to the objective lens assembly (142), the optical stage, the sample stage (170), or a combination thereof, to move flow cell (110) on sample stage (170) relative to the objective lens assembly (142) to provide proper focusing for the imaging operation. In the present example, the focus component (162) utilizes a focus tracking module (160) that is configured to detect a displacement of the objective lens assembly (142) relative to a portion of the flow cell (110) and output data indicative of an in-focus position to the focus component (162) or a component thereof or operable to control the focus component (162), such as controller (120), to move the objective lens assembly (142) to position the corresponding portion of the flow cell (110) in focus of the objective lens assembly (142). By way of example only, focus tracking module (160) may be constructed and operable in accordance with at least some of the teachings of U.S. Pat. No. 10,416,428, entitled "Systems and Methods for Improved Focus Tracking Using a Light Source Configuration," issued September 17, 2019; U.S. Prov. Appln. No. 63/300,531, entitled "Dynamic Detilt Focus Tracking," filed January 18, 2022; or U.S. Prov. Appln. No. 63/410,961, entitled "Spot Error Handling for Focus Tracking," filed September 28, 2022.

In some implementations, an actuator of the focus component (162) or for the sample stage (170) may be physically coupled to objective lens assembly (142), the optical stage, the sample stage (170), or a combination thereof ,such as, for example, by mechanical, magnetic, fluidic, or other attachment or contact directly or indirectly to or with the stage or a component thereof. The actuator of the focus component (162) may be configured to move the objective lens assembly (142) in the z-direction while maintaining the sample stage (170) in the same plane (e.g., maintaining a level or horizontal attitude, perpendicular to the optical axis). In some implementations, the sample stage (170) includes an X direction actuator and a Y direction actuator to form an X-Y stage. The sample stage (170) may also be configured to include one or more tip or tilt actuators to tip or tilt sample stage (170) and/or a portion thereof, such as a flow cell chuck. This may be done, for example, so that flow cell (110) may be leveled dynamically to account for any slope in its surfaces.

Camera system (140) may include one or more image sensors to monitor and track the imaging (e.g., sequencing) of flow cell (110). Camera system (140) may be implemented, for example, as a CCD or CMOS image sensor camera, but other image sensor technologies (e.g., active pixel sensor) may be used. By way of further example only, camera system (140) may include a dual-sensor time delay integration (TDI) camera, a single-sensor camera, a camera with one or more two-dimensional image sensors, and/or other kinds of camera technologies. While camera system (140) and associated optical components are shown as being positioned above flow cell (110) in FIG. 1, one or more image sensors or other camera components may be incorporated into system (100) in numerous other ways as will be apparent to those skilled in the art in view of the teachings herein. For instance, one or more image sensors may be positioned under flow cell (110), such as within the sample stage (170) or below the sample stage (170); or may even be integrated into flow cell (110).

### II. Examples of Flow Cell Structures

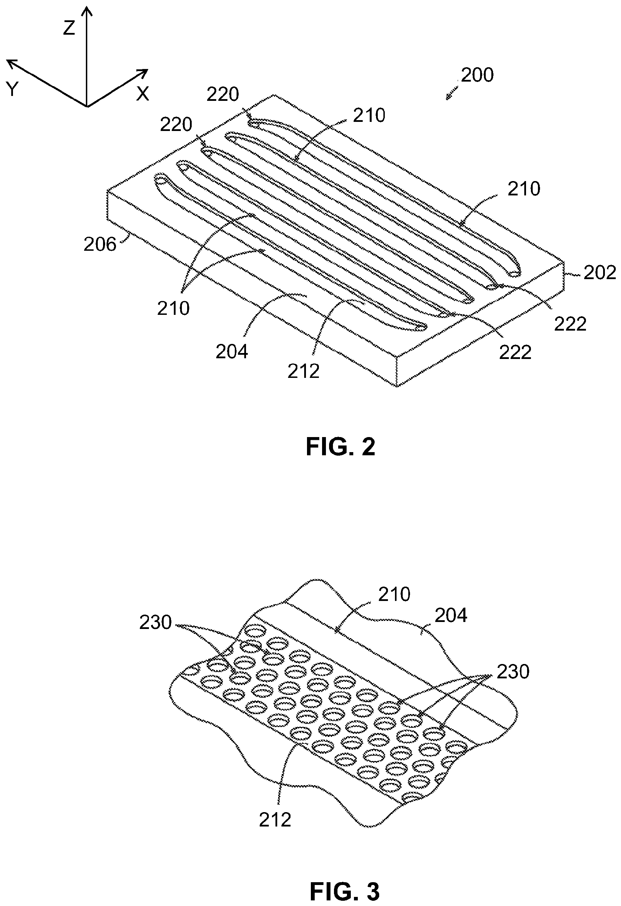

FIGS. 2-3 depict an example of a form that may be taken by flow cell (110). In particular, FIGS. 2-3 show an example of a flow cell (200) that includes a body (202) defining a plurality of elongate flow channels (210), which may be formed in one or more of a bottom surface (206) or an upper surface (204) and/or may be formed by one or more of the bottom surface (206), upper surface (204), one or more interposed layers, and/or one or more adhesive layers structured into a stacked configuration to form the body (202). Flow channels (210) are generally parallel with each other and extend along substantially the entire length of body (302) in the present example. However, in other implementations, the flow channels (210) may be radially positioned relative to each other, perpendicular to each other, and/or positioned in any other angular relationship in substantially the same plane. While five flow channels (210) are shown, a flow cell (200) may include any other suitable number of flow channels (210), including more or fewer than five flow channels (210), such as two flow channels (210), four flow channels (210), eight flow channels (210), etc. The flow cell (200) of this example also includes a set of inlet ports (220) and a set of outlet ports (222), with each port (220, 222) being associated with a corresponding flow channel (210). Thus, each inlet port (220) may be utilized to communicate fluids (e.g., reagents, etc.) to the corresponding channel (210); while each outlet port (222) may be utilized to communicate fluids from the corresponding flow channel (210). In some implementations, two or more flow channels (210) may be fluidically connected by a connection channel such that the two or more flow channels (210) utilize a single inlet port (220) and outlet port (222). In some implementations, the inlet port (220) and outlet port (222) may be positioned and formed within opposing ends of the flow cell (200), may be positioned and formed within substantially the same end of the flow cell (200), and/or may be positioned and formed within any other fixed position of the flow cell (200).

Flow channels (210) of the flow cell (200) may receive reagent fluids from fluid delivery module (190), which may be fluidly coupled with reagents stored in one or more consumable reagent containers (not shown). In addition, or in the alternative, flow channels (210) may be coupled with various other fluid sources or reservoirs, etc. As another illustrative variation, some versions of a consumable cartridge may be configured to removably receive or otherwise integrate the flow cell (200) into a consumable cartridge (not shown) that is removably seated in sample stage (170). In some such versions, flow channels (210) of flow cell (200) may receive fluids from reagent volumes (not shown) via the inlet ports (220). Alternatively, flow cell (200) may be incorporated into system (100) in any other suitable fashion.

FIG. 3 shows a flow channel (210) of flow cell (200) in greater detail. As shown, flow channel (210) includes a plurality of wells (230) formed in a base surface (212) of flow channel (210). By way of example only, each well (230) may be configured to contain nucleic acid strands or other oligonucleotides and thereby provide a reaction site for SBS and/or for other kinds of processes. In some versions, each well (230) has a cylindraceous configuration, with a generally circular cross-sectional profile. In some other versions, each well (230) has a polygonal (e.g., hexagonal, octagonal, square, rectangular, elliptical, etc.) cross-sectional profile. Alternatively, wells (230) may have any other suitable configuration. It should also be understood that wells (230) may be arranged in any suitable pattern, including but not limited to a grid pattern.

### III. Examples of Channel Configurations and Processes for Dynamic Optical System Calibration

In various processes in which system (100) may be used (e.g., SBS processes, etc.), it may be useful to maintain the imaging assembly (122) in a substantially calibrated position to capture suitable images of reaction sites in flow cell (110, 200). To the extent that a calibration routine is initiated at the beginning of a SBS process (or other process), such as at an initial tile or swath of a flow cell (110, 200), such calibration may be less accurate at later positions, such as later tiles or swaths, of the SBS process (or other process). For instance, heat and/or other conditions may affect structural characteristics of one or more features of imaging assembly (122) and/or flow cell (110, 200) through the duration of the SBS process, such as through radiative heating by laser illumination, or other processes. The accuracy of the calibration of the system may be improved despite the heat-induced effects (or other environmental effects) by dynamic re-calibration of imaging assembly (122) to ensure that the images captured by imaging assembly (122) remain suitable through the rest of the SBS process (or other process).

Factors other than heat or other environmental factors may also impact calibration and therefore provide contexts in which dynamic re-calibration may be beneficially applied. For example, errors of a position encoder for sample stage (170) and/or an encoder for a focus component (162), such as a z-stage, may accumulate over time or distance traveled and may result in drift that may affect the calibration of the system. Similarly, in some cases, implementations of other error correction techniques may result in mechanical changes to an imaging assembly that could impact calibration. For example, in some cases, an imaging assembly may be configured to compensate for differences in coverslip thickness through movement of a physical imaging component such as a zoom lens. In other cases (or in cases where an imaging assembly is configured to make mathematical changes to compensate for coverslip thickness), an imaging assembly may be configured to correct for astigmatism through manipulation of compensation plates, such as described in U.S. published patent application 2023/0108792, filed September 30, 2022 for "Apparatus and Methods for Transmitting Light". In these types of cases, dynamic re-calibration such as described herein may be used to compensate for unintended impacts of mechanical changes caused by other error correction techniques implemented in an imaging assembly (e.g., dynamic re-calibration may be used to change the position of an objective lens to compensate for impacts of zoom lens or compensation plate movement). The improvements provided by dynamic re-calibration may increase with the sensitivity of imaging assembly (122). For instance, increases in the sensitivity of imaging assembly (122) may lead to increases in susceptibility of imaging assembly (122) to thermally induced effects or other accumulation errors. In addition, or in the alternative, increases in the sensitivity of imaging assembly (122) may simply make thermally induced effects or accumulation errors more apparent than they otherwise might be in cases of less sensitive imaging assemblies (122).

Successive static calibration methods of the imaging assembly (122) and/or sample stage (170) may increase the overall processing or turnaround time, particularly if an SBS process (or other process) is interrupted to execute one or more calibration routines after the SBS process (or other process) has begun. For instance, in some cases, an SBS process may provide a certain sequence of movements of imaging assembly (122) relative to flow cell (110, 200) (and/or a certain sequence of movements of flow cell (110, 200) relative to imaging assembly (122)). An example of such a sequence of movements may include imaging assembly (122) capturing images or data indicative of illumination intensities of reaction sites in a channel (210) as imaging assembly (122) is moved along the length of channel (210) in the y-direction. In some such versions, imaging assembly (122) may reciprocatingly move in the y-direction over a certain channel (210) a certain number of times (e.g., making several passes along the length of channel (210) for different swaths) before moving in the x-direction to begin imaging reaction sites in the next channel (210).

If such an SBS process has been initiated, and a static calibration routine is executed after the SBS process has been initiated, a static calibration routine may interrupt the certain sequence of movements of imaging assembly (122) relative to flow cell (110, 200) (and/or a certain sequence of movements of flow cell (110, 200) relative to imaging assembly (122)) that is carried out during performance of the SBS process. It may therefore be desirable to provide an alternative, in-motion calibration routine that may be performed in such a way that the calibration routine does not increase the overall time needed to perform an SBS process. Moreover, it may be desirable to provide an alternative calibration routine that may be performed dynamically, throughout performance of an SBS process, to account for any thermal expansions, deformations, or other structural effects that might occur during performance of an SBS process due to increases in heat or other environmental conditions and/or to correct for potential accumulated errors over the course of the scanning process, with such dynamic calibration not interrupting the imaging process that would be normally carried out as part of the SBS process.

### A. Example of Flow Cell with Calibration Regions

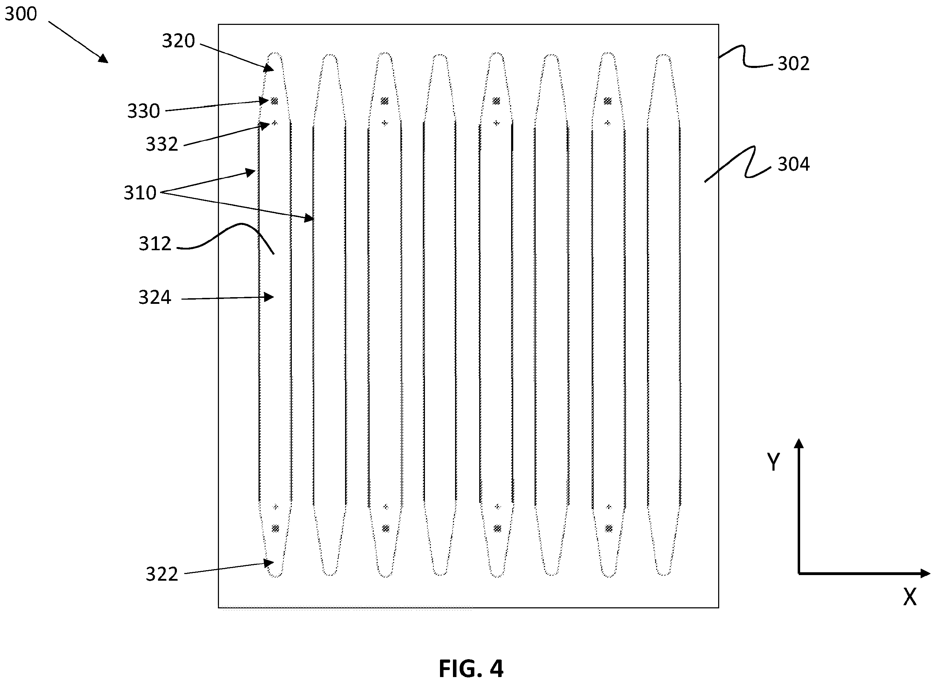

FIG. 4 shows an example of a flow cell (300) that may be used to provide dynamic calibration of imaging assembly (122) during performance of an SBS process (or other process). In other words, flow cell (300) may be used in a process to enable controller (120) to acquire focus calibration characteristics (such as a z-position for the focus component (162) for one or more surfaces of a flow channel) while the sample stage (170) and/or imaging assembly (122) is in motion or to account for thermally induced changes and/or other changes in imaging assembly (122) and/or flow cell (300) in real time, to thereby increase the quality of images captured by imaging assembly (122) through the duration of an SBS process (or other process). Flow cell (300) represents another example of a form that may be taken by flow cell (110, 200). Except as otherwise described below, flow cell (300) of this example may be configured and operable like flow cell (200) described above. Flow cell (300) of this example includes eight flow channels (310). Each flow channel (310) may be formed in one or more of a bottom surface or an upper surface (304) and/or may be formed by one or more of the bottom surface, upper surface (304), one or more interposed layers, and/or one or more adhesive layers structured into a stacked configuration of the body (302) of flow cell (300). Flow channels (310) are generally parallel with each other and extend along substantially the entire length of body (302). While flow cell (300) of this example has eight flow channels (310), any other suitable number of flow channels (310) may be provided, such as one flow channel (310), two flow channels (310), three flow channels (310), four flow channels (310), five flow channels (310), six flow channels (310), seven flow channels (310), or more than eight flow channels (310).

Each channel (310) includes a first end (320), a second end (322), and an intermediate region (324) extending along a length between ends (320, 322). As shown in FIG. 4, this length extends in the y-direction in this example. While not shown in FIGS. 4-5, each channel (310) may include a plurality of wells (230) or other structural features providing reaction sites. In some versions, such wells or other structural features providing reaction sites are only positioned along intermediate region (324). In some other versions, such wells or other structural features providing reaction sites extend all the way to ends (320, 322). In either case, the wells or other structural features providing reaction sites in channels (310) may include nucleic acid strands or other oligonucleotides for SBS and/or for other kinds of processes.

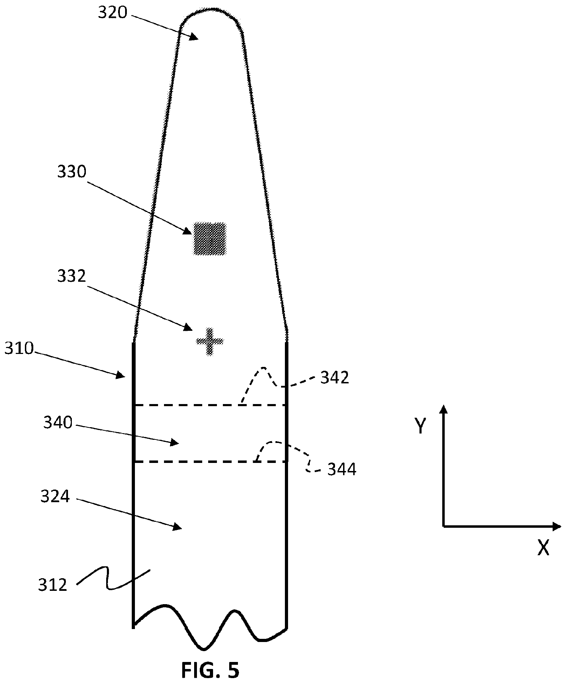

In the present example, a pair of alignment features (330, 332) are provided near each end (320, 322) of alternating channels (310). In some other versions, alignment features (330, 332) are provided in all channels (310), at only one end (320 or 322), for only one channel (310), or in any other suitable arrangement. As best seen in FIG. 5, alignment feature (330) is in the shape of a square; while alignment feature (332) is in the shape of a plus sign or cross. Alternatively, alignment features (330, 332) may take any other suitable form. Alignment feature (332) may be used for X-Y alignment calibration, such as for alignment of a known pattern of a flow cell having patterned features to a loaded flow cell in the system. Alignment feature (330) may be configured to serve as an optical fiducial that promotes optical alignment between imaging assembly (122) and flow cell (110). For instance, imaging assembly (122) may capture an image of flow cell (110). Controller (120) may identify alignment features (330, 332) in the captured image. Based on the identified position of alignment features (330, 332) and/or characteristics of the alignment features (330, 332) in the captured image, the position of one or more features of imaging assembly (122) and/or the position of flow cell (110) may be adjusted to provide appropriate optical alignment between imaging assembly (122) and flow cell (110) and/or to match a known position of a known pattern of a flow cell having patterned features to a loaded flow cell in the system. In addition, or in the alternative, subsequent image processing may be adjusted based on the identified position of alignment features (330, 332) in the captured image. Alternatively, optical alignment may be provided in any other suitable fashion.

As shown in FIG. 5, a calibration region (340) is defined by a first boundary (342) in channel (310) and a second boundary (344) in channel (310). Calibration region (340) is positioned between intermediate region (324) and first end (320). It should be understood that a second calibration region may be positioned between intermediate region (324) and second end (322), with the second calibration region being configured and used like calibration region (340). Thus, intermediate region (324) may extend between two calibration regions (340). Alternatively, only the second calibration region may be utilized in lieu of the first calibration region (340). As shown in FIG. 5, alignment features (330, 332) are positioned between calibration region (340) and first end (320). The particular configuration of calibration region (340) may be varied from what is shown in FIG. 5. For example, one or more of the alignment features (330, 332) may be incorporated into calibration region (340). In other instances, first boundary (342) may be moved closer to first end (320) or further away from first end (320). Similarly, second boundary (344) may be moved closer to first end (320) or further away from first end (320). It should also be understood that a second calibration region may be positioned between intermediate region (324) and second end (322), with the second calibration region being configured and used like calibration region (340).

As noted above, channels (310) include wells or other structural features providing reaction sites; and such wells or other structural features providing reaction sites may include nucleic acid strands or other oligonucleotides for SBS processes and/or for other kinds of processes. In the present example, such wells or other structural features providing reaction sites extend along the length of each calibration region (340). Thus, each calibration region (340) includes wells or other structural features providing reaction sites that may include nucleic acid strands or other oligonucleotides for SBS processes and/or for other kinds of processes. The intermediate region (324) of each channel (310) also includes wells or other structural features providing reaction sites that may include nucleic acid strands or other oligonucleotides for SBS processes and/or for other kinds of processes. Thus, in some versions, the structural configuration, and the presence of nucleic acid strands or other oligonucleotides is the same throughout intermediate region (324) and both calibration regions (340) of each channel (310).

### B. Example of Calibration Process

As noted above, in some cases, an SBS process may provide a certain sequence of movements of imaging assembly (122) relative to flow cell (110, 200, 300) (and/or a certain sequence of movements of flow cell (110, 200, 300) relative to imaging assembly (122)). Depending on the magnitude of the acceleration initiating this relative movement, the structural configuration of system (100), and/or other factors, there may be some degree of shaking or vibration of imaging assembly (122) and/or flow cell (110, 200, 300) at the initial stage of relative movement between imaging assembly (122) and flow cell (110, 200, 300). In some cases, this shaking or vibration may adversely affect the quality of images captured by imaging assembly (122) at the initial stage of relative movement between imaging assembly (122) and flow cell (110, 200, 300). Thus, in some conventional SBS systems, there may be a tendency to either avoid capturing images, or effectively ignore captured images, at the initial stage of relative movement between imaging assembly (122) and flow cell (110, 200, 300). In such scenarios, the regions near the ends (220, 222, 320, 322) of the channels (210, 310) of a flow cell (110, 200, 300), including those associated with the longitudinal positions of calibration region (340), may be disregarded regions such that images of samples in those regions are not utilized. However, in the present example, system (100) will advantageously utilize focus tracking data captured during movement through the calibration region (340) to determine or update a focus model to be applied for the system (100) as the imaging assembly (122) scans a swath of a flow channel (310) of the flow cell (300). Such focus tracking data may be used to facilitate calibration of imaging assembly (122) as described below.

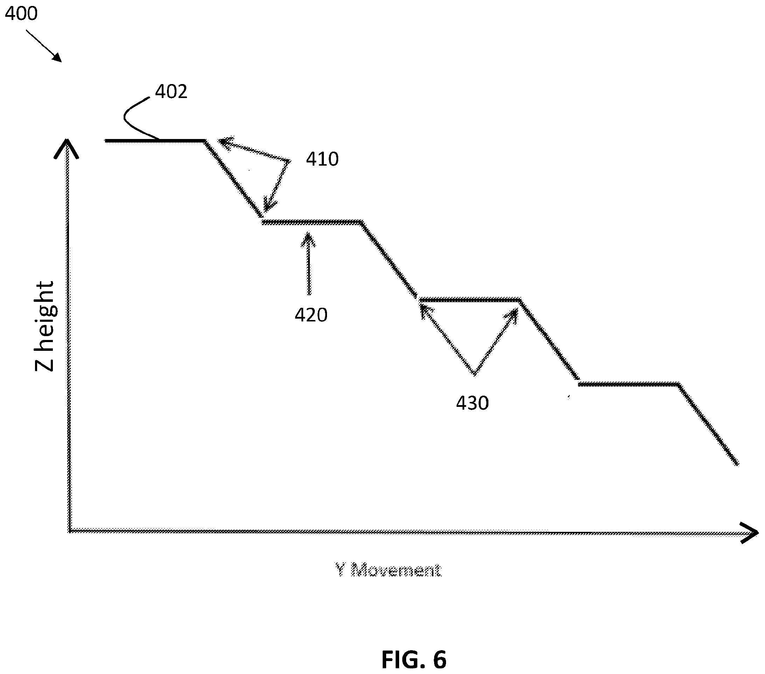

FIG. 6 shows an example of how focus tracking module (160) and other features of imaging assembly (122) may be used to provide an initial static calibration by capturing images at different height positions for a focus component (162). In particular, FIG. 6 shows a graph (400) depicting a plot (402) representing the depth of image capture ("z-height") as a function of longitudinal position ("y-movement") along channel (310). Each horizontal line (420) in plot (402) represents an image capture period by imaging assembly (122), with images being captured between points (430) where the z-height remains constant while the y-position changes. In other instances, the y-position of such points (430) may remain substantially the same. It should be understood that the z and y directions represented in the axes of graph (400) correspond to the z and y directions represented in FIGS. 1, 2, and 4-5. In describing the motion represented by plot (402), the following will describe movement of flow cell (300) relative to imaging assembly (122) along the y-axis. Such movement of flow cell (300) relative to imaging assembly (122) may be driven by one or more actuators of sample stage (170). Some other versions may provide similar relative motion by moving imaging assembly (122) relative to flow cell (300) along the y-axis.

As shown in FIG. 6, a first image (or set of images) may be captured at a first z-height. In some instances, the first images are captured while the imaging assembly (122) or sample stage (170) remains static. In other instances, imaging assembly (122) may move through a first range of y-movement along channel (310). Then, a second image (or set of images) may be captured at a second z-height, after the z-height changes between points (410). The change in z-height between points (410) may be achieved by moving objective lens assembly (142) relative to flow cell (300) along the z-axis, such as via using a z-stage motion controller for focus component (162). In some implementations, the z-stage may comprise a voice coil actuator. Alternatively, the change in z-height between points (410) may be achieved by moving flow cell relative to objective lens assembly (142) along the z-axis. In either scenario, and as shown in FIG. 6, flow cell (300) may move relative to imaging assembly (122) along the y-axis during the change in z-height between points (410). In other instances, the flow cell (300) may remain stationary relative to imaging assembly (122) during the change in z-height. This process may continue until the desired images (or image sets) are captured at the desired z-heights, either as flow cell (300) moves relative to imaging assembly (122) along the y-axis or remains at a substantially static y-axis position. In the example depicted in FIG. 6, there are four discrete images (or four discrete image sets) that are captured at four discrete z-heights.

After the images (or image sets) are captured as described above with reference to FIG. 6, the images may be processed (e.g., by controller (120)) to determine which images (or image sets) provide the best focus for a surface of interest, such as using conventional image processing techniques. The z-height associated with the images (or image sets) providing the best focus may then be utilized for subsequent imaging that is used as part of the SBS process (or other process involving nucleotides, etc., at the reaction sites in channels (310)). Thus, the process described above with reference to FIG. 6 may be used to initially calibrate imaging assembly (122).

In some systems, this calibration may be performed as a separate process that begins and ends before the SBS process (or other process) is initiated; or interrupts the SBS process to be performed. In some such cases, the process described above with reference to FIG. 6 may be carried out over the entire length of channel (310) and/or may be reiterated several times before the entire calibration process is complete. The SBS process may thus need to be delayed until the entire calibration process is complete. To the extent that the SBS process eventually affects imaging assembly (122), flow cell (300), and/or other components of system (100) (e.g., due to thermal expansion, etc.), and the effects warrant re-calibration, a conventional process may provide interruption of the SBS process to perform re-calibration, such that the SBS process may not begin again until the re-calibration is complete. This may substantially increase overall processing time, such that a system operator may be forced to choose between providing optimized calibration or optimized processing time. Alternatively, if environmental factors, such as thermal effects, or accumulation errors, such as position encoder errors, increase beyond a predetermined threshold value, then the initial calibration may be inaccurate and the resulting imaging data may be less accurate, require increased post-acquisition processing, and/or portions of the imaging data may fall below a predetermined quality threshold.

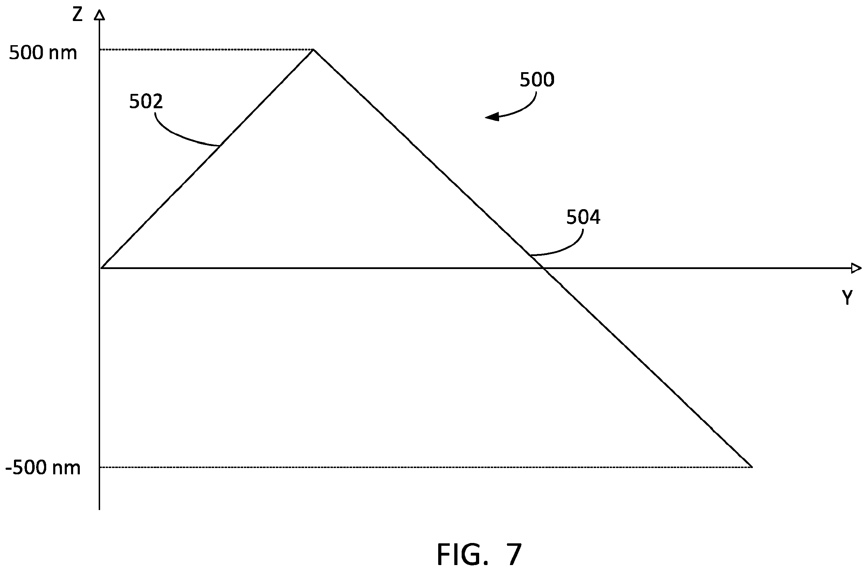

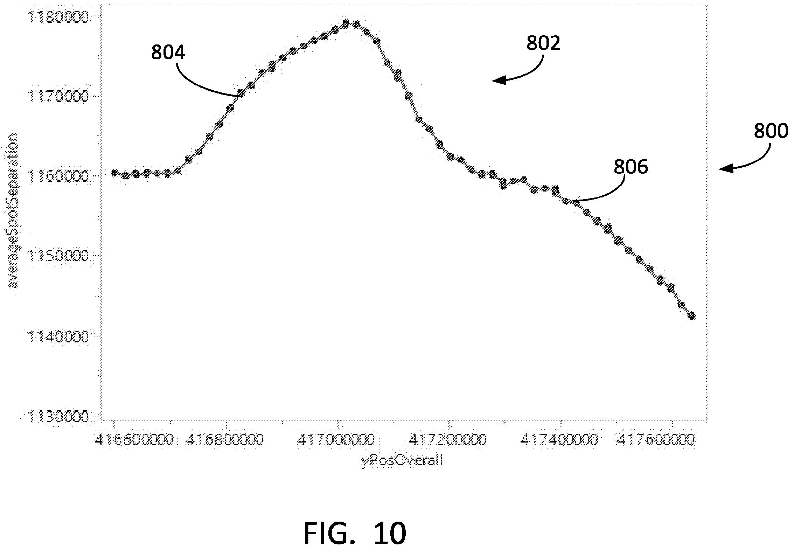

FIG. 7 depicts a motion profile (500) of how focus tracking module (160) and other features of imaging assembly (122) may be used to provide dynamic, in motion, calibration by capturing focus tracking data continuously at different height positions for a focus component (162). In the example shown, a controller of the focus component (162) may be commanded to move the focus component from an initial position to a first position, such as +500 nanometers (nm) as shown, during a first motion (502) and then to a second position, such as -500 nm as shown, during a second motion (504). During this time, the sample stage (170) and/or imaging assembly (122) may continuously move along in the Y-direction through the calibration region (340). As will be discussed in relation to FIGS. 10-12, a y-position encoder or other position tracking element for the sample stage (170) and/or imaging assembly (122) may track a y-position relative to the z-position of the focus component (162). In some implementations, the tracking of the y-position may include outputting the y-position data value to a log at predetermined intervals (e.g., clock cycles).

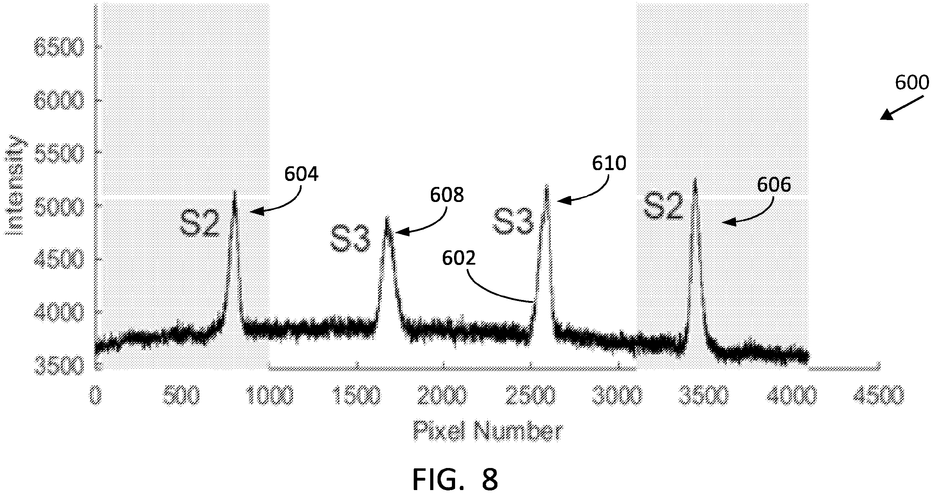

During the movement shown in graph (500) of FIG. 7, a focus tracking module sensor may receive focus tracking data, such as that shown in graph (600) of FIG. 8. In an implementation, focus tracking module (160) or a feature of focus component (162) may utilize a focus tracking illumination source to project a spot through imaging assembly (122) toward the flow cell (300). In some instances, one or more beam splitters may be implemented to split the projected spot into two or more spots toward the flow cell (300). As the flow cell (300) comprises several interfaces between surfaces the projected spot(s) may be reflected by such interfaces of the different surfaces. In an example implementation, a flow cell may include a first surface interface (S1, not shown) where the outer surface of a top substrate material reflects the projected spot, a second surface interface (S2) where the interior surface of the top substrate and an inner fluidic or other material positioned within the flow channel reflects the projected spot, a third surface interface (S3) where the interior surface of a bottom substrate and an inner fluidic or other material positioned within the flow channel reflects the projected spot, and a fourth surface interface (S4, not shown) where the outer surface of a bottom substrate material reflects the projected spot. As shown in FIG. 8, a sensor of the focus component (162) may be configured to detect the illumination intensity of the reflected spots from the interfaces. In the implementation shown, a pair of focus tracking spots are used and the detected illumination data of reflected spots (604, 606, 608, 610) for the second surface interface (S2) and third surface interface (S3) may be utilized to determine an x-axis position, represented by pixel numbers, of each of the reflected spots (604, 606, 608, 610).

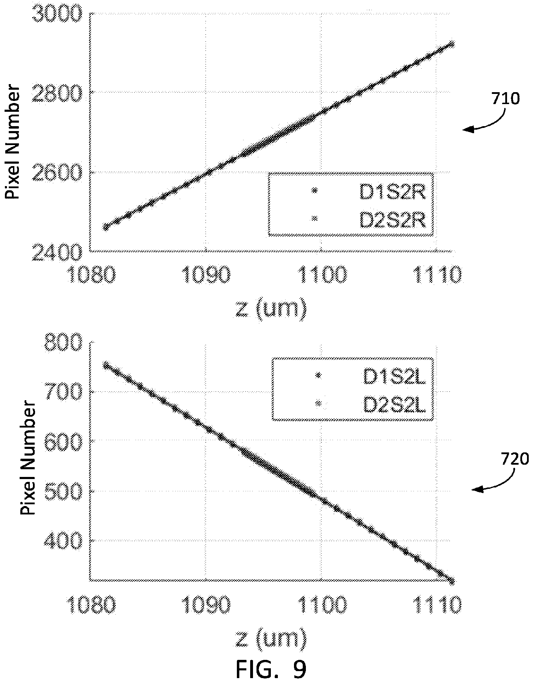

Referring now to FIG. 9, as the z-height of the objective lens (142) of the imaging assembly (122) is changed during the first motion (502) or second motion (504), the x-position of the detected illumination data of the reflected spots changes in a substantially linear relationship to the z-height of the objective lens shown by graphs (710, 720). As shown in FIG. 9, at a first z-height value of 1090 micrometers, a pair of spots reflected from the same surface (shown as S2L and S2R) have a smaller spot separation relative to the same pair of spots reflected from the same surface at a second z-height value of 1110 micrometers. Although the z-height is shown as increasing in value, the orientation of the z-axis in the present graphs is relative to a 0 value datum where the objective lens (142) is further away from the flow cell (300) and increasing values for the z-height move the objective lens (142) closer to the flow cell (300).

In some implementations, a series of average spot separation values may be acquired as the sample stage (170) and/or imaging assembly (122) continuously moves along in the Y-direction through the calibration region (340) and may be correlated with a corresponding y-position encoder value or other position tracking element for the sample stage (170) and/or imaging assembly (122) as shown by graph (800) of FIG. 10. The curve (802) shown in graph (800) may substantially correspond to the motion profile (500) shown in FIG. 7. As shown, the curve (802) has a first portion (804) corresponding to the first motion (502) and a second portion (806) corresponding to the second motion (504).

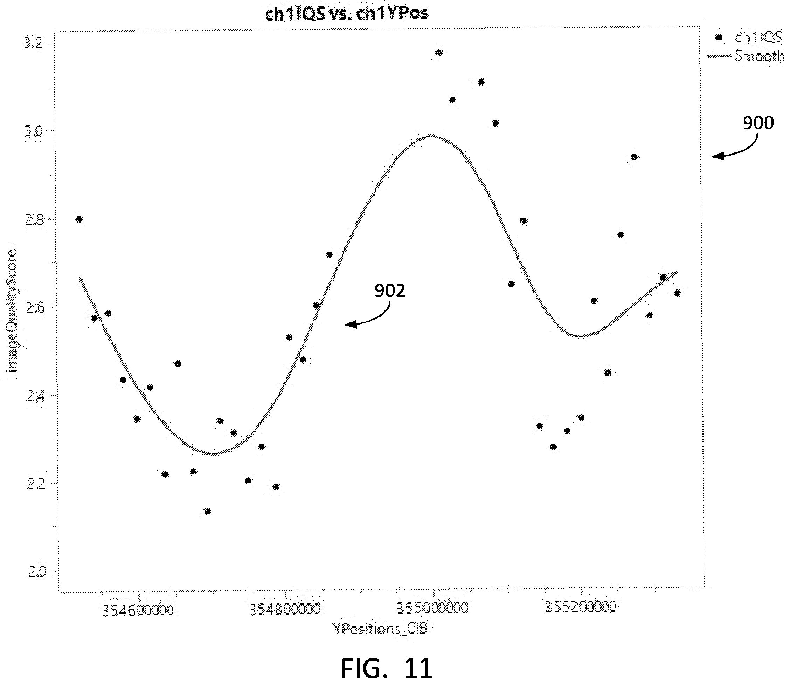

During the acquisition of the series of average spot separation values during the movement through calibration region (340) shown in FIG. 10, the system (100) may also acquire a series of data indicative of image quality, such as an image quality score, at each y-position encoder value as the series of average spot separation values are acquired, such as shown in FIG. 11. In some implementations, these may occur by synchronizing acquisition of the two different systems. For example, a clock cycle of the acquisition system for the average sport separation values may be used for the acquisition system for the data indicative of the image quality. In some implementations, the acquisition system for the average spot separation values may comprise a first printed circuit board assembly (PCBA) and may include a first FPGA; and the acquisition system for the data indicative of the image quality may comprise a second printed circuit board assembly (PCBA) and may include a second FPGA. As shown in the graph (900) in FIG. 11, several data points indicative of image quality at corresponding y-positions within the calibration region (340) are acquired and a smoothed curve fit (902) may be applied to the data. While an image quality score is shown as used, any other data indicative of image quality, such as a Brenner score, may be used.

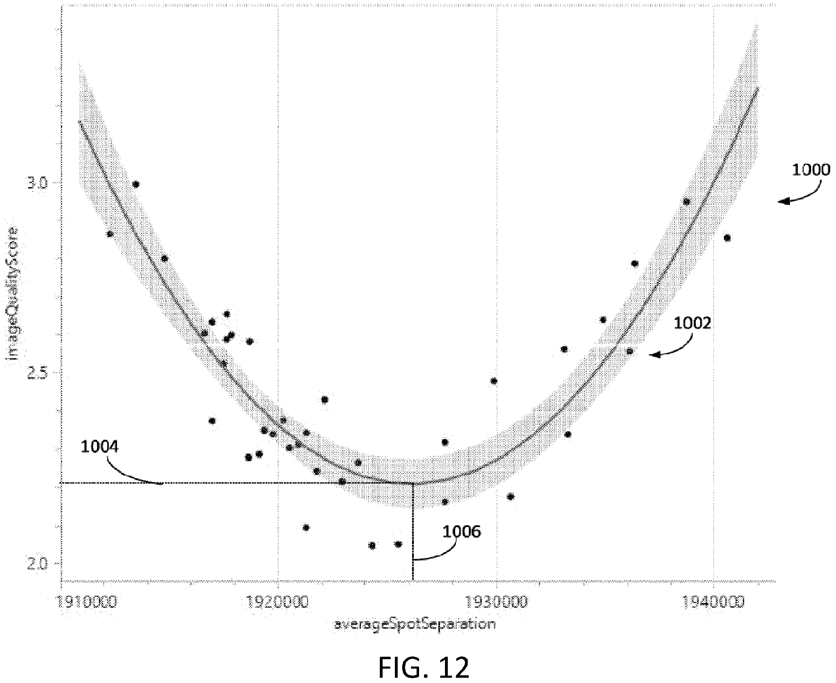

FIG. 12 depicts a graph (1000) showing the correlation of the data points indicative of image quality, such as an image quality score, relative to the data points for the series of average spot separations at the same y-positions. In the present example, the lower an image quality score, the better the image quality for images acquired by the imaging assembly (122) of the system (100). A parabolic curve fit (1002) may be applied to the data points and a minimum for the image quality scores (1004) of the present example of the curve fit (1002) may be identified and the corresponding average spot separation value (1006) may also be identified. In some implementations, the average spot separation value (1006) may be used directly for the z-height position for the focus component (162) for a subsequent imaging acquisition cycle to position the imaging assembly (122); or a specific z-height position value may be identified if spot separation values are not used. In implementations where higher values are indicative of better image quality, a maximum value may instead be identified.

While the foregoing depictions in FIGS. 7-12 show graphical depictions of the different values calculated at different points by different components of the system (100) to determine a z-height position or other value indicative thereof for improved image quality, it should be understood that the values may be directly calculated and implemented without any graphical output.

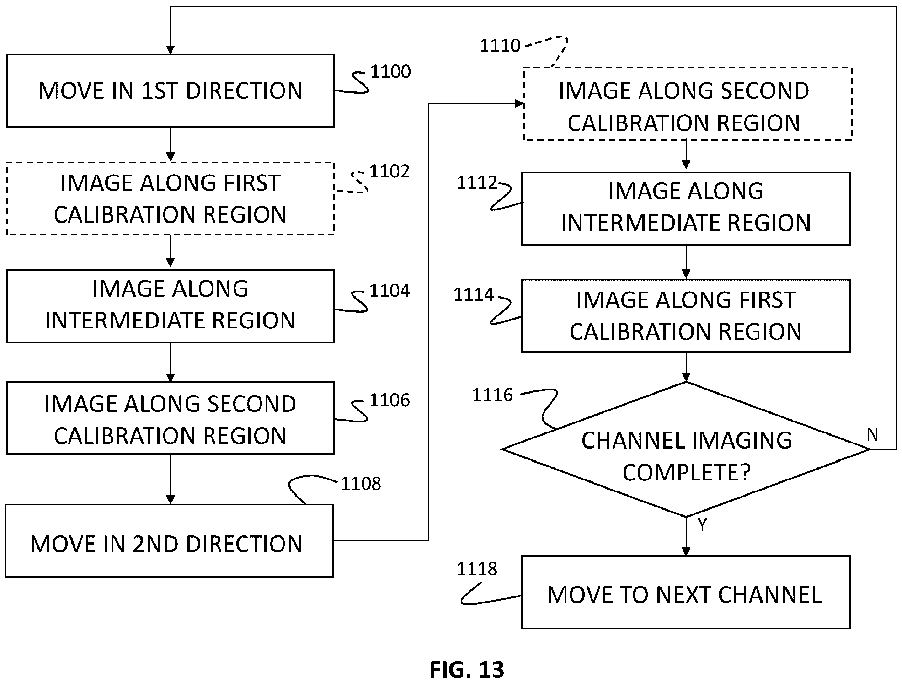

FIG. 13 shows a process that may be employed using a flow cell like flow cell (300), where calibration may be performed dynamically, during the SBS process, such that the calibration may remain optimized in real time without meaningfully increasing the overall time needed to obtain the SBS results. The process shown in FIG. 13 may begin with imaging assembly (122) positioned over first end (320). Flow cell (300) may then move in a first direction relative to imaging assembly (122), along the y-axis, as shown in block (1100). As flow cell (300) moves in this first direction relative to imaging assembly (122), along the y-axis, the field of view of imaging assembly (122) may be effectively moving toward second end (322). In some versions, as represented by the broken line rendering of block (1102) in FIG. 7, imaging assembly (122) may capture images and focus tracking data along the calibration region (340) near first end (320) while simultaneously moving an objective lens of the imaging assembly (122) through a range of z-heights as flow cell (300) moves along the y-axis. In some versions, this step represented by block (1102) may be omitted. If the step represented by block (1102) is carried out, the integrated through focus calibration may be performed in accordance with the teachings provided above in the context of FIGS. 7-12. The z-position and focus model generated by the integrated through focus calibration may then be used for the imaging of block (1104). In other implementations, a static focus model generation process may occur prior to block (1100) and be used for the imaging of block (1104).

Regardless of whether the step represented by block (1102) is carried out as the calibration region (340) near first end (320) passes through the field of view of imaging assembly (122), imaging assembly (122) may capture images of intermediate region (324) of channel (310), as shown in block (1104). These images of intermediate region (324) of channel (310) may be the same kind of images that are captured during conventional SBS processes (e.g., to identify nucleotides at reaction sites in channels (310)). As flow cell (300) continues moving in the first direction relative to imaging assembly (122), along the y-axis, the calibration region (340) near second end (322) eventually reaches the field of view of imaging assembly (122). As the calibration region (340) near second end (322) passes through the field of view of imaging assembly (122), imaging assembly (122) may capture images and perform an integrated through focus calibration during calibration region (340) near second end (322), as shown in block (1106). This integrated through focus calibration during calibration region (340) near second end (322) may be performed in accordance with the teachings provided above in the context of FIGS. 7-12. The data generated by the integrated through focus calibration of block (1106) may then be used for updating a focus model for a subsequent swath and/or a subsequent cycle for the imaging of block (1104) and or imaging of block (1112).

After calibration region (340) near second end (322) has passed through the field of view of imaging assembly (122) (or after the desired integrated through focus calibration is performed for calibration region (340) near second end (322)), the flow cell (300) may be shifted incrementally to a new swath and the movement of flow cell (300) may be reversed. In other words, flow cell (300) may be moved in a second direction relative to imaging assembly (122), along the y-axis, as shown in block (1108). As flow cell (300) moves in this second direction relative to imaging assembly (122), along the y-axis, the field of view of imaging assembly (122) may be effectively moving back toward first end (320). In some versions, as represented by the broken line rendering of block (1110) in FIG. 7, imaging assembly (122) may capture integrated through focus calibration data along the calibration region (340) near second end (322), as flow cell (300) moves along the y-axis. In some other versions, this step represented by block (1110) may be omitted. If the step represented by block (1110) is carried out, the integrated through focus calibration may be performed in accordance with the teachings provided above in the context of FIGS. 7-12.

Regardless of whether the step represented by block (1110) is carried out as the calibration region (340) near second end (322) passes through the field of view of imaging assembly (122), imaging assembly (122) may capture additional images of intermediate region (324) of channel (310), as shown in block (1112). These images of intermediate region (324) of channel (310) may be the same kind of images that are captured during conventional SBS processes (e.g., to identify nucleotides at reaction sites in channels (310)). As flow cell (300) continues moving in the second direction relative to imaging assembly (122), along the y-axis, the calibration region (340) near first end (320) eventually reaches the field of view of imaging assembly (122). As the calibration region (340) near first end (320) passes through the field of view of imaging assembly (122), imaging assembly (122) may perform an integrated through focus calibration in calibration region (340) near first end (320), as shown in block (1114). This integrated through focus calibration performed in calibration region (340) near first end (320) may be performed in accordance with the teachings provided above in the context of FIGS. 7-12.

In the present example, the SBS imaging process may include one or more passes of the same channel (310) under imaging assembly (122). As described above, this may include at least two passes-one in the first direction along the y-axis and another in the second direction along the y-axis. In some versions, the SBS imaging process provides more than two passes of each channel (310) under imaging assembly (122). In other implementations, a single pass per channel (310) may be performed. Thus, the method depicted in FIG. 13 further includes a determination stage, represented by block (1116), to determine whether the imaging of the channel (310) at hand is complete. If the imaging of the channel (310) at hand is not yet complete, then the above-described process may be repeated for that channel (310). By way of example only, some versions may require each channel (310) to be imaged four times before moving onto the next channel (310).

In any case, if the determination stage, represented by block (1116), results in a determination that imaging of the channel (310) at hand is in fact complete, then the process may move to the next channel (310) as represented by block (1118). To move to the next channel (310), flow cell (300) may be moved relative to imaging assembly (122) along the x-y plane. As noted above, such movement may be provided by one or more actuators of sample stage (170). Alternatively, imaging assembly (122) may be moved relative to flow cell (300) along the x-y plane. In either scenario, once the appropriate end (320, 322) of the next channel (310) is within the field of view of imaging assembly (122), the process described above with reference FIG. 13 may be carried out along that next channel (310). This may be repeated until all channels (310) have been imaged.

It should be understood from the foregoing that calibration imaging steps may be seamlessly integrated with SBS imaging steps, such that SBS imaging need not be delayed or interrupted to provide calibration imaging. Similarly, it should be understood from the foregoing that calibration data may be captured through the same continuous, uninterrupted movement of flow cell (300) relative to imaging assembly (122) along the x-y plane. Thus, the capture of calibration data need not impose any meaningful delay on completion of an SBS process. Moreover, the calibration method described above with reference to FIG. 13 may effectively provide a feedback loop with real-time calibration data, thereby minimizing any adverse impact of thermal deformation, drift, and/or other phenomena that might occur during an SBS process and that might otherwise adversely affect SBS images.

In the example described above with reference to FIG. 13, one or both of the two calibration regions (340) of each and every channel (310) of flow cell (300) is/are used for calibration purposes. In some other variations, only the calibration region(s) of every other channel (310) is used for calibration purposes. Alternatively, any other suitable number of calibration regions (340) within a flow cell (300) may be used for calibration purposes. In some further implementations, calibration regions (340) may be positioned between two intermediate regions (324) such that calibration may occur during one or more intermediate positions as the flow channel (310) is imaged in the same direction. For example, such calibration regions may be implemented in flow cells with extended length in the y-direction. In other implementations, calibration regions (340) may be implemented for radial flow cells with radial flow channels (310) or for spiral flow channels (310), such as for wafer based sequencing methods.

Controller (120) may provide various kinds of calibration responses based on calibration data acquired through integrated through focus process as described above. By way of example only, such calibration responses may include adjusting the position and/or orientation of one or more movable components within imaging assembly (122). For instance, controller (120) may provide adjusted z-position of objective lens assembly (142) during acquisition of SBS images along intermediate region (340) (as represented by blocks (504, 512)), based on calibration data acquired through the integrated through focus process as described above. Similarly, controller (120) may provide adjusted z-position ranges of objective lens assembly (142) during acquisition of subsequent integrated through focus processes (as represented by blocks (502, 506, 510, 514)), based on calibration data acquired through calibration as described above. Controller (120) may also adjust how SBS images are processed, based on calibration data acquired through calibration images as described above. Controller (120) may also adjust the irradiance profile (e.g., adjust the intensity of the excitation light) and/or other properties of light emitting assembly (150), based on calibration data acquired through calibration images as described above. Alternatively, controller (120) may provide any other suitable kind(s) of calibration responses based on calibration data acquired through calibration images as described above, in addition to or in lieu of providing the calibration responses outlined above.

**In** versions where camera system (140) includes a **TDI** camera, the calibration routine may determine which spot separation provides the best focus. It should also be understood that the z-movement profile represented in plot (502) is just an example. Other forms of z-movement profiles may include stairstep-shaped profiles, sinusoidal-shaped profiles, or other kinds of profile shapes.

In some variations, the illumination intensity may be enhanced during the calibration imaging. In other words, light emitting assembly (150) may illuminate channel (310) with higher intensity during acquisition of calibration images (as represented by blocks (502, 506, 510, 514)) than the illumination intensity provided acquisition of SBS images (as represented by blocks (504, 512)). Also in some variations, system (100) may intentionally induce physical perturbations to one or more components of imaging assembly (122) and/or flow cell (300) during the integrated through focus process. The data obtained during such induced physical perturbations may further enhance the calibration data.

In the example described above, each calibration region (340) of channel (310) contains nucleotides, just like intermediate region (324) of channel (310). Imaging such nucleotides in calibration region (340) for purposes of calibration may be particularly desirable since nucleotides are also positioned along intermediate region (324), such that the same kind of visual target is used for calibration imaging for the integrated through focus process as is used for SBS imaging. However, some other variations may provide other kinds of visual features in calibration regions (340). For instance, calibration regions (340) may include two-dimensional calibration patterns, three-dimensional calibration structures with known topologies, etc. Another variation of calibration region (340) may extend all the way to the respective end (320, 322) of channel (310), such that first boundary (342) may be effectively eliminated. In some such versions, alignment features (330, 332) may be effectively formed by the absence of nucleotides in channel (310).

In addition to providing real-time accounting for changes that may occur to imaging assembly (122) and/or flow cell (300) during an SBS process (e.g., due to thermal deformation or drift, etc.), the above-described calibration method may also account for localized spatial variations within system (100). For instance, by providing calibration regions (340) at various locations along the x-y plane of flow cell (300), the calibration process may effectively account for tilting, tipping, curving, or other structural variations of the flow cell (300). Thus, the calibration routine executed by controller (120) may be tuned to be sensitive to calibration data that varies as a function of x-y positioning; and thereby apply the corresponding calibration responses as a function of x-y positioning.

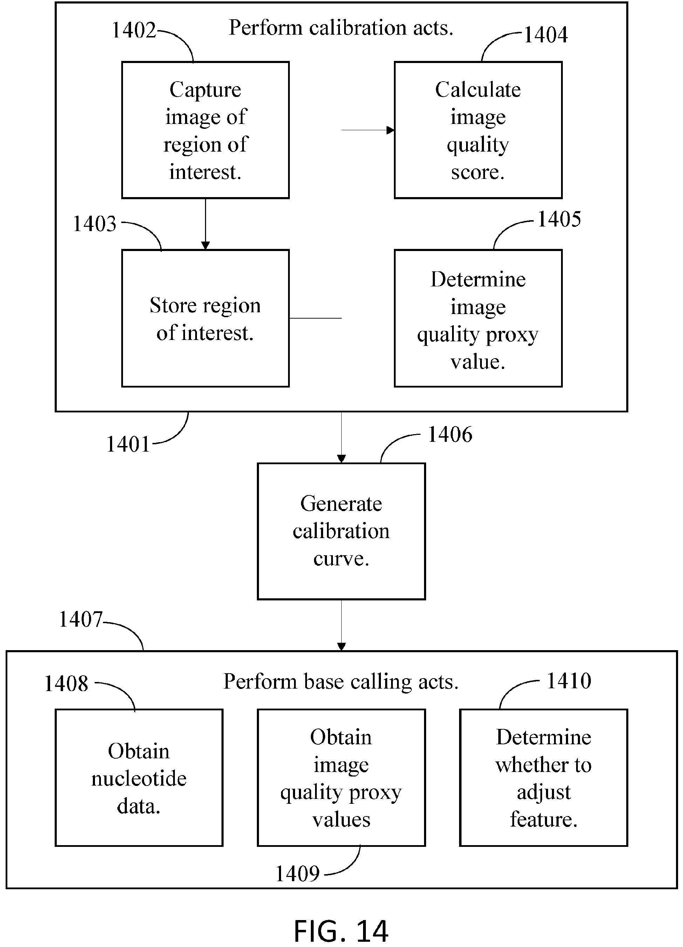

As an example of another type of variation on how the teachings of this disclosure may be implemented, consider FIG. 14, which depicts another flow chart representing an example of a method of dynamically calibrating optical system components. As will be understood by those of ordinary skill in the art, the logic circuitry of a system for biological or chemical analysis such as illustrated in FIG. 1 may be configured with a method such as shown in FIG. 14. However, such a method may also be performed, in whole or in part, using other components, such as external processors or computers which may process data after it is generated using a system such as shown in FIG. 1. Accordingly, the description of the method of FIG. 14 in the context of a system such as shown in FIG. 1 should be understood as being illustrative only, and should not be treated as being limiting.

Turning now to FIG. 14, in the method shown in that figure, a set of calibration acts would be performed in block (1401). These calibration acts may include capturing an image of a region of interest in block (1402). In a method such as shown in FIG. 14, this region of interest may be a two dimensional region on a surface of a channel in a flow cell, which includes a plurality of reaction sites separated from each other along both the length and the width of the channel. Once the image of the region of interest has been captured, it may be stored in a first memory in block (1403) and, in block (1404) an image quality score may be determined for the region of interest. This may be done, for example using contrast gradients in the image of the region of interest (e.g., the higher the contrast, the less blurry the image, and the higher its quality would therefore be assumed to be), or other types of score determination such as Brenner scores such as described above in the context of FIG. 12 may also be used. Simultaneously, one or more image quality proxy values may be determined for that region of interest in block (1405). This may be done by capturing the image of the region of interest over time (e.g., by capturing it row by row as a field of view of the imaging apparatus moves along the length of the flow cell channel), while the image was being captured, capturing spot separation values such as described previously in the context of FIG. 12 and treating the average of those spot separation values as the image quality proxy value for the region of interest.

After these calibration acts had been performed for each region of interest in a plurality of regions of interest, the image quality scores and image quality proxy values for those regions of interest could be used in block (1406) to generate a calibration curve relating image quality proxy values to image quality scores. The parabolic curve illustrated in FIG. 12 is an example of such a calibration curve, and the generation of such a curve may be achieved by fitting a second degree polynomial to coordinates defined by the image quality proxy values and images quality scores. This calibration curve may then be applied in block (1407) to dynamically update the focus of the imaging apparatus while performing a set of base calling acts. These base calling acts may include obtaining nucleotide data in block (1408), which may be performed by using an imaging assembly to detect light emitted from reactants positioned at reaction sites on the surface of the channel, such as described previously in the context of the camera system (140) of FIG. 1 detecting emitted fluorescence from a sample of interest. At the same time, image quality proxy values may be obtained in block (1409) showing how those values change during imaging. Those image quality proxy values could then be used continuously during base calling to determine in block (1410) whether a feature of the imaging assembly (e.g., distance between an objective lens and the surface of the channel) should be adjusted. This may be done, for example, by projecting spots slightly ahead of the region being imaged, determining where the separation between those spots falls on the calibration curve, and, if those spots indicated that an image captured at the location of the spots would be out of focus, adjusting the imaging assembly to address that issue (e.g., by moving the objective lens closer to, or farther from, the surface of the channel).

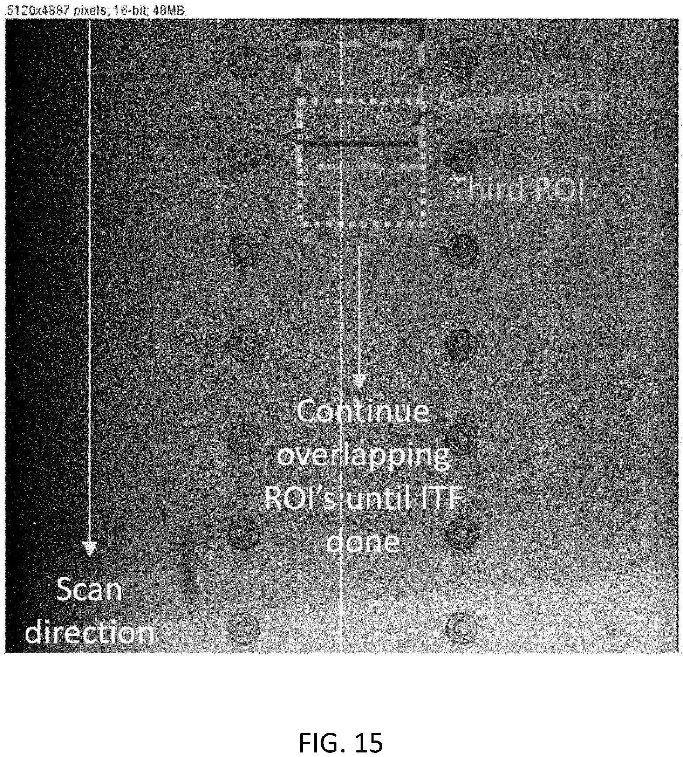

While the above discussion of FIG. 14 indicated how the method represented by the flow chart of that figure could be performed, it should be understood that the example implementation is intended to be illustrative, and that there are many variations on how methods represented by the flow chart of FIG. 14 could be implemented. To illustrate, consider capturing regions of interest in block (1402), and the relationships between those regions. In some implementations, the regions of interest may be adjacent regions of interest, or may be regions of interest which are separated from each other by some distance. However, in other implementations, regions of interest may instead be overlapping regions of interest such as the first region of interest (ROI), second ROI and third ROI illustrated in FIG. 15. For example, in a case where the separation between an objective lens and the surface of the channel being imaged follows a motion profile as shown in FIG. 7 as the field of view of the imaging assembly moves down the length of the channel, regions of interest may be overlapped to so that image quality proxy values (e.g., average spot separation values) transition more smoothly from one region of interest to another, and therefore provide a smoother calibration curve.

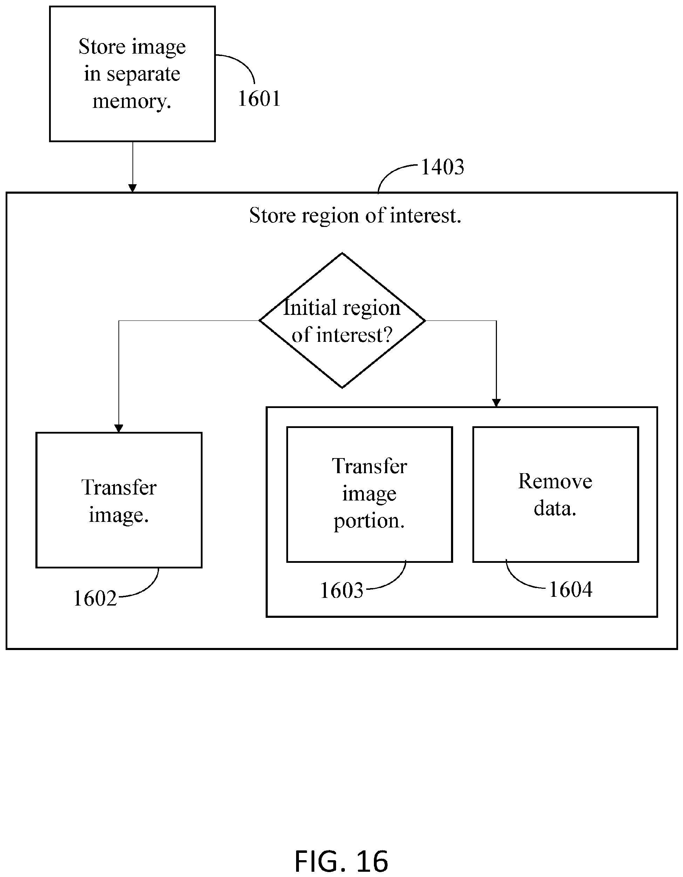

As another example of a type of variation on how methods reflected in the flow chart of FIG. 14 may be implemented, consider the physical devices used in performing the method. For instance, in some cases, a method such as discussed in the context of FIG. 14 may be implemented in an analysis system with logic circuitry that includes both a programmed general purpose processor, as well as a processor-less special purpose logic circuit (e.g., a FPGA). In such a case, the particular capabilities of the different aspects of the logic circuitry may be leveraged to optimize the performance of the method. An example of this type of optimization is provided in FIG. 16, which illustrates a particular approach which may be taken when performing calibration acts such as described previously in the context of block (1401).

In the method shown in FIG. 16, prior to the region of interest being stored as described previously in block (1403) the image of the region of interest would be stored in a separate memory in block (1601). For example, the image of a region of interest may be stored in memory accessible via a program stored in firmware, before being moved to the memory of a FPGA, where it could be processed using the FPGA's faster processing speed to generate an image quality score fast enough for the set of calibration acts to be performed continuously during scanning of a flow cell channel without requiring the scanning of the flow cell channel to be slowed or stopped. Thus, in this type of implementation, after the region of interest is stored in block (1601), it may be stored in a different memory in block (1403) by performing steps which include transferring the region of interest from one memory to the other.

This transfer may also be optimized in some cases. To illustrate, consider a case in which the regions of interest overlap one another and in which the memory in which they are stored in block (1403) is structured as a circular buffer. In such a case, for the first region of interest, storing the region of interest in block (1403) may be performed simply by transferring the image of the region of interest from the separate memory in block (1602). Alternatively, if the region of interest is not the first region of interest, then the transfer may comprise transferring a portion of the region of interest in block (1603), where the portion transferred, when combined with another portion which was already stored in the memory (e.g., FPGA memory) would combine to provide the image of the region of interest. Additionally, data which was already stored may be removed so that it could be replaced by the transferred portion of the image of the first region of interest (e.g., if the transferred portion consisted of 32 rows of a 512 x 512 pixel region of interest, then the oldest 32 rows stored in the memory could be removed when the new 32 rows are transferred in).

As another example, consider a case in which the imaging assembly captures more data than would be used for creating a calibration curve. This may be the case where, for example, the calibration curve is created using image quality scores derived from 512 x 512 pixel regions of interest, but the imaging assembly captures data which has an extent across the width of the channel which is greater than 512 pixels. In such a case, when the image is stored in block (1601), the entire extent of the image may be stored, for example, to maximize the data available if the image was later to be used for sequencing by synthesis. However, only the portion which actually corresponded to the region of interest may be stored in the memory of the processor-less special purpose logic circuit, reflecting the fact that that logic would be particularly used for generating quality scores, and so storing all collected data in that memory may tax its capabilities to no purpose.

Implementations of a method such as that represented by the flow chart of FIG. 14 may also vary from one another in the relationship of the sets of calibration and base calling acts. For example, in some cases, the calibration acts may be performed in first and second calibration regions (e.g., first and second end regions of a channel) in the same manner as described previously in the context of block (1102), block (1106), block (1110) and block (1114) of FIG. 13. However, it is also possible that the calibration acts and/or the generation of the calibration curve may be performed while obtaining nucleotide data during base calling. For instance, as noted above, in some implementations (e.g., where a FPGA with a circular memory buffer is used for storing and deriving image quality scores for regions of interest) the calibration acts may be performed quickly enough to not interfere with scanning a channel. In such an implementation, image quality proxy values obtained during base calling may be used to continuously generate the calibration curve by updating it on an ongoing basis. To facilitate this, in some cases features of an imaging assembly may be continuously varied through a small range (e.g., dithered) around the expected optimal value during base calling, thereby providing a greater variety of data for the ongoing generation of a calibration curve.

Other types of variations are also possible. For example, while in some cases adjustments may be made to a feature of an imaging assembly based on average separation values of a pair of spots, in other cases additional spots (e.g., an additional pair of spots resulting in a square configuration with spots at the vertices) may be used to gather additional data for image optimization. As another example, different implementations may determine whether to make and/or make adjustments at different frequencies. For instance, adjustments may be made on every run, every cycle, every swath, or every tile during sequencing. As another example, in some implementations, focus information collected on one pass may be used in other passes. For instance, the first time scanning the surface of a channel (e.g., when imaging a first swath), an image profile of the channel may be created. On subsequent scanning cycles for that channel, that profile may be used to control scanning speed by slowing down on areas where the profile indicated there was a sharp slope, or speeding up on areas where the profile indicated there was a gradual (or no) slope. Further variations are also possible, and will be immediately apparent to those of skill in the art in light of this disclosure. Accordingly, the examples provided herein, as well as the variations on those examples, should be understood as being illustrative only, and should not be treated as implying limitations on the protection provided by this document or any related document.

### IV. Examples of Combinations

The following examples relate to various non-exhaustive ways in which the teachings herein may be combined or applied. The following examples are not intended to restrict the coverage of any claims that may be presented at any time in this application or in subsequent filings of this application. No disclaimer is intended. The following examples are being provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.

### Example 1 (not claimed)

An apparatus, comprising: a flow cell including: a channel, the channel having a first end region, a second end region, and an intermediate region extending between the first end region and the second end region, the channel defining a length that includes the first end region, intermediate region, and the second end region, the channel being configured to receive a fluid, and the channel including one or more calibration regions in the first end region, the second end region, or the intermediate region, a plurality of reaction sites positioned along the intermediate region, each reaction site being configured to contain a biological sample carried by the fluid, each reaction site being further configured to receive an excitation light; an imaging assembly operable to receive light emitted from a reactant positioned at the reaction sites in response to the excitation light; and a processor, the processor being configured to: drive relative movement between at least a portion of the imaging assembly and the flow cell along a continuous range of motion to thereby enable the imaging assembly to capture images along the length of the channel, activate the imaging assembly to capture one or more calibration images for the one or more calibration regions, during a first portion of the continuous range of motion, and activate the imaging assembly to capture images of the reaction sites during a second portion of the continuous range of motion.

### Example 2 (not claimed)

The apparatus of Example 1, wherein the one or more calibration regions is positioned in the first end region.

### Example 3 (not claimed)

The apparatus of Example 1 or 2, wherein the one or more calibration regions is positioned in the second end region.

### Example 4 (not claimed)

apparatus of any one of Examples 1-3, wherein the one or more calibration regions is positioned in the intermediate region.

### Example 5 (not claimed)

The apparatus of any of Examples 1-4, the one or more calibration regions include nucleotides.

### Example 6 (not claimed)

The apparatus of any of Examples 1-5, the processor being further configured to adjust a feature of the imaging assembly based at least in part on data from the one or more calibration images.

### Example 7 (not claimed)

A method comprising: communicating fluid through a channel of a flow cell; moving at least a portion of an imaging assembly relative to the flow cell through a range of motion; and while moving the at least a portion of the imaging assembly relative to the flow cell through the range of motion: capturing one or more calibration images of a first calibration region via the imaging assembly, the first calibration region being positioned in a first end region of the channel, and capturing one or more images of reaction sites via the imaging assembly, the reaction sites being positioned at an intermediate region of the channel.

### Example 8 (not claimed)

The method of Example 7, further comprising, while moving the at least a portion of the imaging assembly relative to the flow cell through the range of motion, capturing one or more calibration images of a second calibration region via the imaging assembly, the second calibration region being positioned at a second end region of the channel.

### Example 9 (not claimed)

The method of Example 7 or 8, further comprising adjusting a feature of the imaging assembly based at least in part on data from the one or more calibration images.

### Example 10 (not claimed)

The method of any one of Examples 7-9, further comprising, performing sequencing-by-synthesis analysis based on the one or more images of the reaction sites.

### Example 11 (not claimed)

The method of Example 10, the sequencing-by-synthesis analysis being performed while moving the at least a portion of the imaging assembly relative to the flow cell through the range of motion.

### Example 12 (not claimed)

A method comprising: communicating fluid through a channel of a flow cell; performing sequencing-by-synthesis via the flow cell; and while performing sequencing-by-synthesis via the flow cell: capturing one or more calibration images of a first calibration region via an imaging assembly, the first calibration target being positioned at a first end region of the channel, and capturing one or more images of reaction sites via the imaging assembly, the reaction sites being positioned at an intermediate region of the channel.

### Example 13 (not claimed)

The method of Example 12, performing sequencing-by-synthesis via the flow cell including moving at least a portion of the imaging assembly relative to the flow cell through a range of motion.

### Example 14 (not claimed)

The method of Example 13, capturing one or more images of reaction sites via the imaging assembly being performed while moving the at least a portion of the imaging assembly relative to the flow cell through the range of motion.

### Example 15 (not claimed)

The method of Example 14, capturing one or more calibration images of the first calibration region via the imaging assembly being performed while moving the at least a portion of the imaging assembly relative to the flow cell through the range of motion.

### Example 16 (not claimed)

A processor-readable medium including contents that are configured to cause a processor to process data by performing the method of Example 12.

### Example 17

An apparatus comprising: a flow cell comprising one or more channels, wherein each of the one or more channels has a length and a width with the length being greater than the width, and comprises a surface having a plurality of reaction sites; an imaging assembly to receive light emitted from a reactant positioned at the reaction sites in response to an excitation light; a focus component to, for each channel from the one or more channels, obtain image quality proxy values for the surface of that channel; and logic circuitry, wherein the logic circuitry is to, for a subject channel from the one or more channels: for each of a subject plurality of regions of interest, wherein each region of interest is a two dimensional region on the surface of the subject channel having a plurality of reaction sites separated from each other along the length of the subject channel and a plurality of reaction sites separated from each other along the width of the channel, perform a set of calibration acts comprising: capturing, using the imaging assembly, an image of that region of interest; storing the image of that region of interest in a first memory; determining one or more image quality proxy values for that region of interest using the focus component; and calculating an image quality score for that region of interest; generate a calibration curve relating image quality scores for the regions of interest to image quality proxy values for the regions of interest; and while driving relative motion of the subject channel and a field of view of the imaging assembly along the length of the subject channel via one or more actuators of a sample stage on which the flow cell is mounted or via one or more actuators of the imaging assembly, perform a set of base calling acts comprising: obtaining nucleotide data based on using the imaging assembly to detect light emitted from reactants positioned at reaction sites on the surface of the subject channel; obtaining one or more image quality proxy values using the focus component while obtaining nucleotide data; and based on the calibration curve and on the one or more image quality proxy values obtained while obtaining nucleotide data, determining whether to adjust a feature of the imaging assembly; wherein the logic circuitry comprises: a programmed general purpose processor; and processor-less special purpose logic circuit; the first memory is a local memory resident on the processor-less special purpose logic circuit; the apparatus comprises a second memory operatively connected to the programmed general purpose processor; for each region of interest from the subject plurality of regions of interest the set of calibration acts comprises, before storing the image of that region of interest in the first memory, store the image of that region of interest in the second memory; for an initial region of interest from the subject plurality of regions of interest, storing the image of that region of interest in the first memory comprises transferring the image of that region of interest from the second memory to the first memory; for each region of interest from the subject plurality of regions of interest other than the initial region of interest, storing that region of interest in the first memory comprises: transferring a first portion of the image of that region of interest from the second memory to the first memory at a time when the first memory already contains a second portion of that region of interest as a result of that second portion being comprised by a different, previously stored, region of interest, wherein the first portion of the image and that region of interest and the second portion of that region of interest combine to provide the image of the region of interest; and removing data from the first memory, wherein the data removed from the first memory is replaced by the first portion of the image of the region of interest; the processor-less special purpose logic circuit is to, for each region of interest from the plurality of regions of interest, calculate the image quality score for that region of interest.

### Example 18

The apparatus of example 17, wherein for each region of interest from the subject plurality of regions of interest, that region of interest overlaps with at least one other region of interest from the subject plurality of regions of interest along the length of the subject channel.

### Example 19

The apparatus of example 17, wherein, for at least one region of interest from the subject plurality of regions of interest, at least a portion of the second portion that region of interest is comprised by a plurality of different, previously stored, regions of interest.

### Example 20

The apparatus of example 17, wherein, for each of the subject plurality of regions of interest: capturing, using the imaging assembly, the image of that region of interest comprises capturing a corresponding image of the subject channel, wherein: the corresponding image of the subject channel has an extent along the width of the subject channel greater than an extent of the region of interest along the width of the subject channel; and the corresponding image of the subject channel has an extent along the length of the subject channel equal to an extent of the region of interest along the length of the subject channel; and storing the image of that region of interest in the second memory comprises storing the corresponding image of the subject channel in the second memory.

### Example 21

The apparatus of example 17, wherein each of the one or more channels comprises a first end region, a second end region, and an intermediate region extending between the first end region and the second end region; the logic circuitry is to: during a first period, move a field of view of the imaging assembly along the length of the subject channel from the first end region of the subject channel, through the intermediate region of the subject channel, to the second end region of the subject channel; perform the set of calibration acts with a first plurality of regions of interest as the subject plurality of regions of interest during the first period, wherein the first plurality of regions of interest are regions of interest in the first end region of the subject channel; during a second period, move a field of view of the imaging assembly along the length of the subject channel from the second end region of the subject channel, through the intermediate regions of the subject channel, to the first end region of the subject channel; and perform the set of calibration acts with a second plurality of regions as the subject plurality of regions of interest during the second period, wherein the second plurality of regions of interest are regions of interest in the second end region of the subject channel.

### Example 22

The apparatus of example 21, wherein the logic circuitry is to perform the set of calibration acts with a third plurality of regions of interest as the subject plurality of regions of interest, wherein the third plurality of regions of interest are regions of interest in the intermediate region of the subject channel.

### Example 23

The apparatus of example 22, wherein the logic circuitry is to: while performing the set of calibration acts with the first plurality of regions of interest as the subject plurality of regions of interest, drive relative movement between the feature of the imaging assembly and the flow cell along a height, wherein the height is perpendicular to the length and width of the subject channel, through a continuous range of motion between a first value and a second value; and while performing the set of calibration acts with the third plurality of regions of interest as the subject plurality of regions of interest, drive relative movement between the feature of the imaging assembly and the flow cell along the height through a continuous range of motion between a third value and a fourth value, wherein the third value and the fourth value are each between the first value and the second value.

### Example 24

The apparatus of example 17, wherein: the feature of the imaging assembly is an objective lens; the logic circuitry is to, for the subject channel from the one or more channels, while performing the set of calibration acts: drive relative movement between the objective lens and the surface of the subject channel through a continuous range of motion along a height which is perpendicular to the length and the width of the subject channel; and drive relative motion of the subject channel and the field of view of the imaging assembly along the length of the subject channel; the focus component is to, for each channel from the one or more channels, obtain image quality proxy values for the surface of that channel by performing acts comprising projecting a set of spots onto the surface of the channel, and detecting reflections of the set of spots from the surface of that channel; for each region of interest from the subject plurality of regions of interest: the one or more image quality proxy values for that region of interest comprise an average spot separation value for that region of interest; and determining the one or more image quality proxy values for that region of interest using the focus component comprises the focus component projecting the set of spots onto, detecting reflections of the set of spots from, the surface of the subject channel while capturing the image of that region of interest; and determining whether to adjust the feature of the imaging assembly comprises determining whether to adjust relative positions of the objective lens and the surface of the subject channel along the height.

### Example 25

A method comprising: for each of a subject plurality of regions of interest, performing a set of calibration acts, wherein each region of interest is a two dimensional region on a surface of a subject channel of a flow cell, the subject channel having a plurality of reaction sites separated from each other along the length of the subject channel and a plurality of reaction sites separated from each other along the width of the channel, perform a set of calibration acts comprising: capturing, using an imaging assembly, an image of that region of interest; storing the image of that region of interest in a first memory; determining one or more image quality proxy values for that region of interest using a focus component of a system for analyzing chemical or biological materials; calculating an image quality score for that region of interest; generating a calibration curve relating image quality scores for the regions of interest to image quality proxy values for the regions of interest; while driving relative motion of the subject channel and a field of view of the imaging assembly along the length of the subject channel via one or more actuators of a sample stage on which the flow cell is mounted or via one or more actuators of the imaging assembly, perform a set of base calling acts comprising: obtaining nucleotide data based on using the imaging assembly to detect light emitted from reactants positioned at reaction sites on the surface of the subject channel; obtaining one or more image quality proxy values using the focus component while obtaining nucleotide data; and based on the calibration curve and on the one or more image quality proxy values obtained while obtaining nucleotide data, determining whether to adjust a feature of the imaging assembly;
wherein: the first memory is a local memory resident on a processor-less special purpose logic circuit; for each region of interest from the subject plurality of regions of interest the set of calibration acts comprises, before storing the image of that region of interest in the first memory, store the image of that region of interest in a second memory, wherein the second memory is operatively connected to a general purpose processor; for an initial region of interest from the subject plurality of regions of interest, storing the image of that region of interest in the first memory comprises transferring the image of that region of interest from the second memory to the first memory; for each region of interest from the subject plurality of regions of interest other than the initial region of interest, storing that region of interest in the first memory comprises: transferring a first portion of the image of that region of interest from the second memory to the first memory at a time when the first memory already contains a second portion of that region of interest as a result of that second portion being comprised by a different, previously stored, region of interest, wherein the first portion of the image and that region of interest and the second portion of that region of interest combine to provide the image of the region of interest; and removing data from the first memory, wherein the data removed from the first memory is replaced by the first portion of the image of the region of interest; the processor-less special purpose logic circuit is to, for each region of interest from the plurality of regions of interest, calculate the image quality score for that region of interest;
wherein, for at least one region of interest from the subject plurality of regions of interest, at least a portion of the second portion that region of interest is comprised by a plurality of different, previously stored, regions of interest.

### Example 26

The method of example 25, wherein for each region of interest from the subject plurality of regions of interest, that region of interest overlaps with at least one other region of interest from the subject plurality of regions of interest along the length of the subject channel.

### Example 27

The method of example 25, wherein. for each of the subject plurality of regions of interest: capturing, using the imaging assembly, the image of that region of interest comprises capturing a corresponding image of the subject channel, wherein: the corresponding image of the subject channel has an extent along the width of the subject channel greater than an extent of the region of interest along the width of the subject channel; and the corresponding image of the subject channel has an extent along the length of the subject channel equal to an extent of the region of interest along the length of the subject channel; and storing the image of that region of interest in the second memory comprises storing the corresponding image of the subject channel in the second memory.

### Example 28

The method of example 25, wherein: each of the one or more channels comprises a first end region, a second end region, and an intermediate region extending between the first end region and the second end region; the method comprises: during a first period, moving a field of view of the imaging assembly along the length of the subject channel from the first end region of the subject channel, through the intermediate region of the subject channel, to the second end region of the subject channel; performing the set of calibration acts with a first plurality of regions of interest as the subject plurality of regions of interest during the first period, wherein the first plurality of regions of interest are regions of interest in the first end region of the subject channel; during a second period, moving a field of view of the imaging assembly along the length of the subject channel from the second end region of the subject channel, through the intermediate regions of the subject channel, to the first end region of the subject channel; and performing the set of calibration acts with a second plurality of regions as the subject plurality of regions of interest during the second period, wherein the second plurality of regions of interest are regions of interest in the second end region of the subject channel;
wherein the method comprises performing the set of calibration acts with a third plurality of regions of interest as the subject plurality of regions of interest, wherein the third plurality of regions of interest are regions of interest in the intermediate region of the subject channel; and determining a nucleotide sequence for a sample of biological material by performing sequencing by synthesis based on nucleotide data captured from the third plurality of regions of interest.

### Example 29

The method of example 28, wherein the method comprises: while performing the set of calibration acts with the first plurality of regions of interest as the subject plurality of regions of interest, driving relative movement between the feature of the imaging assembly and the flow cell along a height, wherein the height is perpendicular to the length and width of the subject channel, through a continuous range of motion between a first value and a second value; and while performing the set of calibration acts with the third plurality of regions of interest as the subject plurality of regions of interest, driving relative movement between the feature of the imaging assembly and the flow cell along the height through a continuous range of motion between a third value and a fourth value, wherein the third value and the fourth value are each between the first value and the second value.

### Example 30

The method of example 25, wherein: the feature of the imaging assembly is an objective lens; the method comprises for the subject channel, while performing the set of calibration acts: driving relative movement between the objective lens and the surface of the subject channel through a continuous range of motion along a height which is perpendicular to the length and the width of the subject channel; and driving relative motion of the subject channel and the field of view of the imaging assembly along the length of the subject channel; the focus component is to, for the subject channel, obtain image quality proxy values for the surface of that channel by performing acts comprising projecting a set of spots onto the surface of the channel, and detecting reflections of the set of spots from the surface of that channel; for each region of interest from the subject plurality of regions of interest: the one or more image quality proxy values for that region of interest comprise an average spot separation value for that region of interest; and determining the one or more image quality proxy values for that region of interest using the focus component comprises the focus component projecting the set of spots onto, detecting reflections of the set of spots from, the surface of the subject channel while capturing the image of that region of interest; and determining whether to adjust the feature of the imaging assembly comprises determining whether to adjust relative positions of the objective lens and the surface of the subject channel along the height.

### V. Miscellaneous

While the foregoing examples are provided in the context of a system (100) that may be used in nucleotide sequencing processes, the teachings herein may also be readily applied in other contexts, including in systems that perform other processes (i.e., other than nucleotide sequencing procedures). The teachings herein are thus not necessarily limited to systems that are used to perform nucleotide sequencing processes.

It is to be understood that the subject matter described herein is not limited in its application to the details of construction and the arrangement of components set forth in the description herein or illustrated in the drawings hereof. The subject matter described herein is capable of other implementations and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items.

When used in the claims, the term "set" should be understood as one or more things which are grouped together. Similarly, when used in the claims "based on" should be understood as indicating that one thing is determined at least in part by what it is specified as being "based on." Where one thing is required to be exclusively determined by another thing, then that thing will be referred to as being "exclusively based on" that which it is determined by.

Unless specified or limited otherwise, the terms "mounted," "connected," "supported," and "coupled" and variations thereof are used broadly and encompass both direct and indirect mountings, connections, supports, and couplings. Further, "connected" and "coupled" are not restricted to physical or mechanical connections or couplings. Also, it is to be understood that phraseology and terminology used herein with reference to device or element orientation (such as, for example, terms like "above," "below," "front," "rear," "distal," "proximal," and the like) are only used to simplify description of one or more examples described herein, and do not alone indicate or imply that the device or element referred to must have a particular orientation. In addition, terms such as "outer" and "inner" are used herein for purposes of description and are not intended to indicate or imply relative importance or significance.

It is to be understood that the above description is intended to be illustrative, and not restrictive. For example, the above-described examples (and/or aspects thereof) may be used in combination with each other. In addition, many modifications may be made to adapt a particular situation or material to the teachings of the presently described subject matter without departing from its scope. While the dimensions, types of materials and coatings described herein are intended to define the parameters of the disclosed subject matter, they are by no means limiting and instead illustrations. Many further examples will be apparent to those of skill in the art upon reviewing the above description. The scope of the disclosed subject matter should, therefore, be determined with reference to the appended claims. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects.

The following claims recite aspects of certain examples of the disclosed subject matter and are considered to be part of the above disclosure. These aspects may be combined with one another.

## Claims

1. An apparatus, comprising:
a flow cell (300) comprising one or more channels (310), wherein each of the one or more channels has a length and a width with the length being greater than the width, and comprises a surface having a plurality of reaction sites;
an imaging assembly (122)
to receive light emitted from a reactant positioned at the reaction sites in response to an excitation light;
a focus component (162)
to, for each channel from the one or more channels, obtain image quality proxy values for the surface of that channel; and
logic circuitry comprising a first memory, wherein the logic circuitry is configured to, for a subject channel from the one or more channels:
for each of a subject plurality of regions of interest, wherein each region of interest is a two dimensional region on the surface of the subject channel having a plurality of reaction sites separated from each other along the length of the subject channel and a plurality of reaction sites separated from each other along the width of the channel, perform a set of calibration acts comprising:
capturing, using the imaging assembly, an image of that region of interest;
storing the image of that region of interest in a first memory;
determining one or more image quality proxy values for that region of interest using the focus component; and
calculating an image quality score for that region of interest;
generate a calibration curve relating image quality scores for the regions of interest to image quality proxy values for the regions of interest; and
while driving relative motion of the subject channel and a field of view of the imaging assembly along the length of the subject channel via one or more actuators of a sample stage on which the flow cell is mounted or via one or more actuators of the imaging assembly, perform a set of base calling acts comprising:
obtaining nucleotide data based on using the imaging assembly to detect light emitted from reactants positioned at reaction sites on the surface of the subject channel;
obtaining one or more image quality proxy values using the focus component while obtaining nucleotide data; and
based on the calibration curve and on the one or more image quality proxy values obtained while obtaining nucleotide data, determining whether to adjust a feature of the imaging assembly
wherein:
the logic circuitry comprises:
a programmed general purpose processor; and
processor-less special purpose logic circuit;
the first memory is a local memory resident on the processor-less special purpose logic circuit;
the apparatus comprises a second memory operatively connected to the programmed general purpose processor;
for each region of interest from the subject plurality of regions of interest the set of calibration acts comprises, before storing the image of that region of interest in the first memory, store the image of that region of interest in the second memory;
for an initial region of interest from the subject plurality of regions of interest, storing the image of that region of interest in the first memory comprises transferring the image of that region of interest from the second memory to the first memory;
for each region of interest from the subject plurality of regions of interest other than the initial region of interest, storing that region of interest in the first memory comprises:
transferring a first portion of the image of that region of interest from the second memory to the first memory at a time when the first memory already contains a second portion of that region of interest as a result of that second portion being comprised by a different, previously stored, region of interest, wherein the first portion of the image and that region of interest and the second portion of that region of interest combine to provide the image of the region of interest; and
removing data from the first memory, wherein the data removed from the first memory is replaced by the first portion of the image of the region of interest;
the processor-less special purpose logic circuit is configured to, for each region of interest from the plurality of regions of interest, calculate the image quality score for that region of interest.

2. The apparatus of claim 1, wherein for each region of interest from the subject plurality of regions of interest, that region of interest overlaps with at least one other region of interest from the subject plurality of regions of interest along the length of the subject channel.

3. The apparatus of claim 1, wherein, for at least one region of interest from the subject plurality of regions of interest, at least a portion of the second portion that region of interest is comprised by a plurality of different, previously stored, regions of interest.

4. The apparatus of claim 1, wherein, for each of the subject plurality of regions of interest:
capturing, using the imaging assembly (122), the image of that region of interest comprises
capturing a corresponding image of the subject channel, wherein:
the corresponding image of the subject channel has an extent along the width of the subject channel greater than an extent of the region of interest along the width of the subject channel; and
the corresponding image of the subject channel has an extent along the length of the subject channel equal to an extent of the region of interest along the length of the subject channel;
and
storing the image of that region of interest in the second memory comprises storing the corresponding image of the subject channel in the second memory.

5. The apparatus of claim 1, wherein:
each of the one or more channels (210, 310)
comprises a first end region, a second end region, and an intermediate region extending between the first end region and the second end region;
the logic circuitry is configured to:
during a first period, move a field of view of the imaging assembly along the length of the subject channel from the first end region of the subject channel, through the intermediate region of the subject channel, to the second end region of the subject channel;
perform the set of calibration acts with a first plurality of regions of interest as the subject plurality of regions of interest during the first period, wherein the first plurality of regions of interest are regions of interest in the first end region of the subject channel;
during a second period, move a field of view of the imaging assembly along the length of the subject channel from the second end region of the subject channel, through the intermediate regions of the subject channel, to the first end region of the subject channel; and
perform the set of calibration acts with a second plurality of regions as the subject plurality of regions of interest during the second period, wherein the second plurality of regions of interest are regions of interest in the second end region of the subject channel.

6. The apparatus of claim 5, wherein the logic circuitry is configured to perform the set of calibration acts with a third plurality of regions of interest as the subject plurality of regions of interest, wherein the third plurality of regions of interest are regions of interest in the intermediate region of the subject channel.

7. The apparatus of claim 6, wherein the logic circuitry is configured to: while performing the set of calibration acts with the first plurality of regions of interest as the subject plurality of regions of interest, drive relative movement between the feature of the imaging assembly (122) and the flow cell (110) along a height, wherein the height is perpendicular to the length and width of the subject channel, through a continuous range of motion between a first value and a second value; and while performing the set of calibration acts with the third plurality of regions of interest as the subject plurality of regions of interest, drive relative movement between the feature of the imaging assembly (122) and the flow cell (110) along the height through a continuous range of motion between a third value and a fourth value, wherein the third value and the fourth value are each between the first value and the second value.

8. The apparatus of claim 1, wherein: the feature of the imaging assembly (122) is an objective lens (142); the logic circuitry is configured to, for the subject channel from the one or more channels (210, 310), while performing the set of calibration acts:
drive relative movement between the objective lens (142) and the surface of the subject
channel through a continuous range of motion along a height which is perpendicular to the length and the width of the subject channel; and drive relative motion of the subject channel and the field of view of the imaging
assembly (122) along the length of the subject channel;
the focus component (162) is configured to, for each channel from the one or more channels, obtain image quality proxy values for the surface of that channel by performing acts comprising projecting a set of spots onto the surface of the channel, and detecting reflections of the set of spots from the surface of that channel;
for each region of interest from the subject plurality of regions of interest:
the one or more image quality proxy values for that region of interest comprise an average spot separation value for that region of interest; and
determining the one or more image quality proxy values for that region of interest using the focus component comprises the focus component projecting the set of spots onto, detecting reflections of the set of spots from, the surface of the subject channel while capturing the image of that region of interest;
and
determining whether to adjust the feature of the imaging assembly comprises determining whether to adjust relative positions of the objective lens and the surface of the subject channel along the height.

9. A method comprising:
for each of a subject plurality of regions of interest, performing a set of calibration acts, wherein each region of interest is a two dimensional region on a surface of a subject channel of a flow cell (110, 200, 300), the subject channel having a plurality of reaction sites separated from each other along the length of the subject channel and a plurality of reaction sites separated from each other along the width of the channel, perform a set of calibration acts comprising:
capturing, using an imaging assembly (122), an image of that region of interest;
storing the image of that region of interest in a first memory;
determining one or more image quality proxy values for that region of interest using a focus component (162) of a system for analyzing chemical or biological materials;
calculating an image quality score for that region of interest;
generating a calibration curve relating image quality scores for the regions of interest to image quality proxy values for the regions of interest;
while driving relative motion of the subject channel and a field of view of the imaging assembly along the length of the subject channel via one or more actuators of a sample stage on which the flow cell is mounted or via one or more actuators of the imaging assembly, perform a set of base calling acts comprising:
obtaining nucleotide data based on using the imaging assembly to detect light emitted from reactants positioned at reaction sites on the surface of the subject channel;
obtaining one or more image quality proxy values using the focus component while obtaining nucleotide data; and
based on the calibration curve and on the one or more image quality proxy values obtained while obtaining nucleotide data, determining whether to adjust a feature of the imaging assembly;
wherein:
the first memory is a local memory resident on a processor-less special purpose logic circuit;
for each region of interest from the subject plurality of regions of interest the set of calibration acts comprises, before storing the image of that region of interest in the first memory, store the image of that region of interest in a second memory, wherein the second memory is operatively connected to a general purpose processor;
for an initial region of interest from the subject plurality of regions of interest, storing the image of that region of interest in the first memory comprises transferring the image of that region of interest from the second memory to the first memory;
for each region of interest from the subject plurality of regions of interest other than the initial region of interest, storing that region of interest in the first memory comprises:
transferring a first portion of the image of that region of interest from the second memory to the first memory at a time when the first memory already contains a second portion of that region of interest as a result of that second portion being comprised by a different, previously stored, region of interest, wherein the first portion of the image and that region of interest and the second portion of that region of interest combine to provide the image of the region of interest; and
removing data from the first memory, wherein the data removed from the first memory is replaced by the first portion of the image of the region of interest;
the processor-less special purpose logic circuit is to, for each region of interest from the plurality of regions of interest, calculate the image quality score for that region of interest;
for at least one region of interest from the subject plurality of regions of interest, at least a portion of the second portion that region of interest is comprised by a plurality of different, previously stored, regions of interest.

10. The method of claim 9, wherein, for each of the subject plurality of regions of interest:
capturing, using the imaging assembly (122), the image of that region of interest comprises capturing a corresponding image of the subject channel, wherein:
the corresponding image of the subject channel has an extent along the width of the subject channel greater than an extent of the region of interest along the width of the subject channel; and
the corresponding image of the subject channel has an extent along the length of the subject channel equal to an extent of the region of interest along the length of the subject channel;
and
storing the image of that region of interest in the second memory comprises storing the corresponding image of the subject channel in the second memory.

11. The method of claim 9, wherein:
each of the one or more channels (210, 310)
comprises a first end region, a second end region, and an intermediate region extending between the first end region and the second end region;
the method comprises:
during a first period, moving a field of view of the imaging assembly along the length of the subject channel from the first end region of the subject channel, through the intermediate region of the subject channel, to the second end region of the subject channel;
performing the set of calibration acts with a first plurality of regions of interest as the subject plurality of regions of interest during the first period, wherein the first plurality of regions of interest are regions of interest in the first end region of the subject channel;
during a second period, moving a field of view of the imaging assembly along the length of the subject channel from the second end region of the subject channel, through the intermediate regions of the subject channel, to the first end region of the subject channel; and
performing the set of calibration acts with a second plurality of regions as the subject plurality of regions of interest during the second period, wherein the second plurality of regions of interest are regions of interest in the second end region of the subject channel;
performing the set of calibration acts with a third plurality of regions of interest as the subject plurality of regions of interest, wherein the third plurality of regions of interest are regions of interest in the intermediate region of the subject channel; and
determining a nucleotide sequence for a sample of biological material by performing sequencing by synthesis based on nucleotide data captured from the third plurality of regions of interest.

12. The method of claim 11, wherein the method comprises:
while performing the set of calibration acts with the first plurality of regions of interest as the subject plurality of regions of interest, driving relative movement between the feature of the imaging assembly (122) and the flow cell (110, 200, 300) along a height, wherein the height is perpendicular to the length and width of the subject channel, through a continuous range of motion between a first value and a second value; and
while performing the set of calibration acts with the third plurality of regions of interest as the subject plurality of regions of interest, driving relative movement between the feature of the imaging assembly and the flow cell along the height through a continuous range of motion between a third value and a fourth value, wherein the third value and the fourth value are each between the first value and the second value.

13. The method of claim 9, wherein:
the feature of the imaging assembly (122) is an objective lens (142);
the method comprises for the subject channel, while performing the set of calibration acts:
driving relative movement between the objective lens and the surface of the subject channel through a continuous range of motion along a height which is perpendicular to the length and the width of the subject channel; and
driving relative motion of the subject channel and the field of view of the imaging assembly along the length of the subject channel;
the focus component is to, for the subject channel, obtain image quality proxy values for the surface of that channel by performing acts comprising projecting a set of spots onto the surface of the channel, and detecting reflections of the set of spots from the surface of that channel;
for each region of interest from the subject plurality of regions of interest:
the one or more image quality proxy values for that region of interest comprise an average spot separation value for that region of interest; and
determining the one or more image quality proxy values for that region of interest using the focus component comprises the focus component projecting the set of spots onto, detecting reflections of the set of spots from, the surface of the subject channel while capturing the image of that region of interest;
and
determining whether to adjust the feature of the imaging assembly comprises determining whether to adjust relative positions of the objective lens and the surface of the subject channel along the height.

## Patentansprüche

1. Ein Gerät, das Folgendes beinhaltet:
eine Durchflusszelle (300), die einen oder mehrere Kanäle (310) beinhaltet, wobei jeder der einen oder mehreren Kanäle eine Länge und eine Breite aufweist, wobei die Länge größer ist als die Breite, und eine Oberfläche, die eine Vielzahl von Reaktionsstellen aufweist, beinhaltet;
eine Bildgebungsanordnung (122) zum Empfangen von Licht, das von einem an den Reaktionsstellen positionierten Reaktanten als Reaktion auf ein Anregungslicht emittiert wird;
eine Fokuskomponente (162), um für jeden der einen oder mehreren Kanäle Bildqualitätsproxywerte für die Oberfläche dieses Kanals zu erhalten; und
eine Logikschaltung, die einen ersten Speicher beinhaltet, wobei die Logikschaltung konfiguriert ist, um für einen Subjektkanal aus dem einen oder den mehreren Kanälen:
für jede einer Subjektvielzahl von Regionen von Interesse, wobei jede Region von Interesse eine zweidimensionale Region auf der Oberfläche des Subjektkanals ist, die eine Vielzahl von Reaktionsstellen, die entlang der Länge des Subjektkanals voneinander getrennt sind, und eine Vielzahl von Reaktionsstellen, die entlang der Breite des Kanals voneinander getrennt sind, aufweist, einen Satz von Kalibrierungshandlungen durchzuführen, die Folgendes beinhalten:
Erfassen, unter Verwendung der Bildgebungsanordnung, eines Bildes dieser Region von Interesse;
Speichern des Bildes dieser Region von Interesse in einem ersten Speicher;
Bestimmen eines oder mehrerer Bildqualitätsproxywerte für diese Region von Interesse unter Verwendung der Fokuskomponente; und
Berechnen einer Bildqualitätsbewertung für diese Region von Interesse;
Erzeugen einer Kalibrierungskurve, die Bildqualitätsbewertungen für die Regionen von Interesse mit Bildqualitätsproxywerten für die Regionen von Interesse in Beziehung setzt; und
während eine relative Bewegung des Subjektkanals und eines Sichtfelds der Bildgebungsanordnung entlang der Länge des Subjektkanals über einen oder mehrere Aktoren eines Probentischs, auf dem die Durchflusszelle montiert ist, oder über einen oder mehrere Aktoren der Bildgebungsanordnung angetrieben wird, Durchführen eines Satzes von Basenaufrufhandlungen, die Folgendes beinhalten:
Erhalten von Nukleotiddaten basierend auf der Verwendung der Bildgebungsanordnung, um Licht zu detektieren, das von Reaktanten emittiert wird, die an Reaktionsstellen auf der Oberfläche des Subjektkanals positioniert sind;
Erhalten eines oder mehrerer Bildqualitätsproxywerte unter Verwendung der Fokuskomponente, während Nukleotiddaten erhalten werden; und
basierend auf der Kalibrierungskurve und auf dem einen oder den mehreren Bildqualitätsproxywerten, die erhalten werden, während Nukleotiddaten erhalten werden, Bestimmen, ob ein Merkmal der Bildgebungsanordnung angepasst werden soll,
wobei:
die Logikschaltung Folgendes beinhaltet:
einen programmierten Universalprozessor; und
eine prozessorlose Speziallogikschaltung;
der erste Speicher ein lokaler Speicher ist, der sich auf der prozessorlosen Speziallogikschaltung befindet;
das Gerät einen zweiten Speicher beinhaltet, der operativ mit dem programmierten Universalprozessor verbunden ist;
für jede Region von Interesse aus der Subjektvielzahl von Regionen von Interesse der Satz von Kalibrierungshandlungen vor dem Speichern des Bildes dieser Region von Interesse in dem ersten Speicher das Speichern des Bildes dieser Region von Interesse in dem zweiten Speicher beinhaltet;
für eine anfängliche Region von Interesse aus der Subjektvielzahl von Regionen von Interesse das Speichern des Bildes dieser Region von Interesse in dem ersten Speicher das Übertragen des Bildes dieser Region von Interesse von dem zweiten Speicher zu dem ersten Speicher beinhaltet;
für jede Region von Interesse aus der Subjektvielzahl von Regionen von Interesse, die sich von der anfänglichen Region von Interesse unterscheidet, das Speichern dieser Region von Interesse in dem ersten Speicher Folgendes beinhaltet:
Übertragen eines ersten Abschnitts des Bildes dieser Region von Interesse von dem zweiten Speicher zu dem ersten Speicher zu einem Zeitpunkt, zu dem der erste Speicher bereits einen zweiten Abschnitt dieser Region von Interesse enthält, als ein Ergebnis dessen, dass dieser zweite Abschnitt in einer anderen, zuvor gespeicherten Region von Interesse enthalten ist, wobei sich der erste Abschnitt des Bildes und diese Region von Interesse und der zweite Abschnitt dieser Region von Interesse kombinieren, um das Bild der Region von Interesse bereitzustellen; und
Entfernen von Daten aus dem ersten Speicher, wobei die aus dem ersten Speicher entfernten Daten durch den ersten Abschnitt des Bildes der Region von Interesse ersetzt werden;
die prozessorlose Speziallogikschaltung konfiguriert ist, um für jede Region von Interesse aus der Vielzahl von Regionen von Interesse die Bildqualitätsbewertung für diese Region von Interesse zu berechnen.

2. Gerät gemäß Anspruch 1, wobei sich für jede Region von Interesse aus der Subjektvielzahl von Regionen von Interesse diese Region von Interesse mit mindestens einer anderen Region von Interesse aus der Subjektvielzahl von Regionen von Interesse entlang der Länge des Subjektkanals überlappt.

3. Gerät gemäß Anspruch 1, wobei für mindestens eine Region von Interesse aus der Subjektvielzahl von Regionen von Interesse mindestens ein Abschnitt des zweiten Abschnitts dieser Region von Interesse aus einer Vielzahl von unterschiedlichen, zuvor gespeicherten Regionen von Interesse besteht.

4. Gerät gemäß Anspruch 1, wobei für jede der Subjektvielzahl von Regionen von Interesse:
das Erfassen, unter Verwendung der Bildgebungsanordnung (122), des Bildes dieser Region von Interesse das Erfassen eines entsprechenden Bildes des Subjektkanals beinhaltet, wobei:
das entsprechende Bild des Subjektkanals eine Ausdehnung entlang der Breite des Subjektkanals aufweist, die größer als eine Ausdehnung der Region von Interesse entlang der Breite des Subjektkanals ist; und
das entsprechende Bild des Subjektkanals eine Ausdehnung entlang der Länge des Subjektkanals aufweist, die gleich einer Ausdehnung der Region von Interesse entlang der Länge des Subjektkanals ist;
und
das Speichern des Bildes dieser Region von Interesse in dem zweiten Speicher das Speichern des entsprechenden Bildes des Subjektkanals in dem zweiten Speicher beinhaltet.

5. Gerät gemäß Anspruch 1, wobei:
jeder des einen oder der mehreren Kanäle (210, 310) eine erste Endregion, eine zweite Endregion und eine Zwischenregion, die sich zwischen der ersten Endregion und der zweiten Endregion erstreckt, beinhaltet;
die Logikschaltung konfiguriert ist, um:
während einer ersten Periode ein Sichtfeld der Bildgebungsanordnung entlang der Länge des Subjektkanals von der ersten Endregion des Subjektkanals durch die Zwischenregion des Subjektkanals zu der zweiten Endregion des Subjektkanals zu bewegen;
den Satz von Kalibrierungshandlungen mit einer ersten Vielzahl von Regionen von Interesse als die Subjektvielzahl von Regionen von Interesse während der ersten Periode durchzuführen, wobei die erste Vielzahl von Regionen von Interesse Regionen von Interesse in der ersten Endregion des Subjektkanals sind;
während einer zweiten Periode ein Sichtfeld der Bildgebungsanordnung entlang der Länge des Subjektkanals von der zweiten Endregion des Subjektkanals durch die Zwischenregionen des Subjektkanals zu der ersten Endregion des Subjektkanals zu bewegen; und
den Satz von Kalibrierungshandlungen mit einer zweiten Vielzahl von Regionen als die Subjektvielzahl von Regionen von Interesse während der zweiten Periode durchzuführen, wobei die zweite Vielzahl von Regionen von Interesse Regionen von Interesse in der zweiten Endregion des Subjektkanals sind.

6. Gerät gemäß Anspruch 5, wobei die Logikschaltung konfiguriert ist, um den Satz von Kalibrierungshandlungen mit einer dritten Vielzahl von Regionen von Interesse als die Subjektvielzahl von Regionen von Interesse durchzuführen, wobei die dritte Vielzahl von Regionen von Interesse Regionen von Interesse in der Zwischenregion des Subjektkanals sind.

7. Gerät gemäß Anspruch 6, wobei die Logikschaltung konfiguriert ist, um:
während des Durchführens des Satzes von Kalibrierungshandlungen mit der ersten Vielzahl von Regionen von Interesse als die Subjektvielzahl von Regionen von Interesse, eine relative Bewegung zwischen dem Merkmal der Bildgebungsanordnung (122) und der Durchflusszelle (110) entlang einer Höhe, wobei die Höhe senkrecht zu der Länge und Breite des Subjektkanals ist, durch einen kontinuierlichen Bewegungsbereich zwischen einem ersten Wert und einem zweiten Wert anzutreiben; und
während des Durchführens des Satzes von Kalibrierungshandlungen mit der dritten Vielzahl von Regionen von Interesse als die Subjektvielzahl von Regionen von Interesse, eine relative Bewegung zwischen dem Merkmal der Bildgebungsanordnung (122) und der Durchflusszelle (110) entlang der Höhe durch einen kontinuierlichen Bewegungsbereich zwischen einem dritten Wert und einem vierten Wert anzutreiben, wobei der dritte Wert und der vierte Wert jeweils zwischen dem ersten Wert und dem zweiten Wert liegen.

8. Gerät gemäß Anspruch 1, wobei:
das Merkmal der Bildgebungsanordnung (122) eine Objektivlinse (142) ist;
die Logikschaltung konfiguriert ist, um für den Subjektkanal von dem einen oder den mehreren Kanälen (210, 310) während des Durchführens des Satzes von Kalibrierungshandlungen:
eine relative Bewegung zwischen der Objektivlinse (142) und der Oberfläche des Subjektkanals durch einen kontinuierlichen Bewegungsbereich entlang einer Höhe, die senkrecht zu der Länge und der Breite des Subjektkanals ist, anzutreiben; und
eine relative Bewegung des Subjektkanals und des Sichtfelds der Bildgebungsanordnung (122) entlang der Länge des Subjektkanals anzutreiben:
die Fokuskomponente (162) konfiguriert ist, um für jeden Kanal aus dem einen oder den mehreren Kanälen Bildqualitätsproxywerte für die Oberfläche dieses Kanals durch Durchführen von Handlungen zu erhalten, die das Projizieren eines Satzes von Punkten auf die Oberfläche des Kanals und das Detektieren von Reflexionen des Satzes von Punkten von der Oberfläche dieses Kanals beinhalten;
für jede Region von Interesse aus der Subjektvielzahl von Regionen von Interesse:
der eine oder die mehreren Bildqualitätsproxywerte für diese Region von Interesse einen durchschnittlichen Punkttrennungswert für diese Region von Interesse beinhalten; und
das Bestimmen des einen oder der mehreren Bildqualitätsproxywerte für diese Region von Interesse unter Verwendung der Fokuskomponente beinhaltet, dass die Fokuskomponente den Satz von Punkten auf die Oberfläche des Subjektkanals projiziert, Reflexionen des Satzes von Punkten von der Oberfläche des Subjektkanals detektiert, während das Bild dieser Region von Interesse erfasst wird; und
das Bestimmen, ob das Merkmal der Bildgebungsanordnung angepasst werden soll, das Bestimmen beinhaltet, ob relative Positionen der Objektivlinse und der Oberfläche des Subjektkanals entlang der Höhe angepasst werden sollen.

9. Ein Verfahren, das Folgendes beinhaltet:
für jede von einer Subjektvielzahl von Regionen von Interesse, Durchführen eines Satzes von Kalibrierungshandlungen, wobei jede Region von Interesse eine zweidimensionale Region auf einer Oberfläche eines Subjektkanals einer Durchflusszelle (110, 200, 300) ist,
wobei der Subjektkanal eine Vielzahl von Reaktionsstellen, die entlang der Länge des Subjektkanals voneinander getrennt sind, und eine Vielzahl von Reaktionsstellen, die entlang der Breite des Kanals voneinander getrennt sind, aufweist, Durchführen eines Satzes von Kalibrierungshandlungen, die Folgendes beinhalten:
Erfassen, unter Verwendung einer Bildgebungsanordnung (122), eines Bildes dieser Region von Interesse;
Speichern des Bildes dieser Region von Interesse in einem ersten Speicher; Bestimmen eines oder mehrerer Bildqualitätsproxywerte für diese Region von Interesse unter Verwendung einer Fokuskomponente (162) eines Systems zum Analysieren von chemischen oder biologischen Materialien;
Berechnen einer Bildqualitätsbewertung für diese Region von Interesse;
Erzeugen einer Kalibrierungskurve, die Bildqualitätsbewertungen für die Regionen von Interesse mit Bildqualitätsproxywerten für die Regionen von Interesse in Beziehung setzt;
während eine relative Bewegung des Subjektkanals und eines Sichtfelds der Bildgebungsanordnung entlang der Länge des Subjektkanals über einen oder mehrere Aktoren eines Probentischs, auf dem die Durchflusszelle montiert ist, oder über einen oder mehrere Aktoren der Bildgebungsanordnung angetrieben wird, Durchführen eines Satzes von Basenaufrufhandlungen, die Folgendes beinhalten:
Erhalten von Nukleotiddaten basierend auf der Verwendung der Bildgebungsanordnung, um Licht zu detektieren, das von Reaktanten emittiert wird, die an Reaktionsstellen auf der Oberfläche des Subjektkanals positioniert sind;
Erhalten eines oder mehrerer Bildqualitätsproxywerte unter Verwendung der Fokuskomponente, während Nukleotiddaten erhalten werden; und
basierend auf der Kalibrierungskurve und auf dem einen oder den mehreren Bildqualitätsproxywerten, die erhalten werden, während Nukleotiddaten erhalten werden, Bestimmen, ob ein Merkmal der Bildgebungsanordnung angepasst werden soll;
wobei:
der erste Speicher ein lokaler Speicher ist, der sich auf einer prozessorlosen Speziallogikschaltung befindet;
für jede Region von Interesse aus der Subjektvielzahl von Regionen von Interesse der Satz von Kalibrierungshandlungen vor dem Speichern des Bildes dieser Region von Interesse in dem ersten Speicher das Speichern des Bildes dieser Region von Interesse in einem zweiten Speicher beinhaltet, wobei der zweite Speicher operativ mit einem Universalprozessor verbunden ist;
für eine anfängliche Region von Interesse aus der Subjektvielzahl von Regionen von Interesse das Speichern des Bildes dieser Region von Interesse in dem ersten Speicher das Übertragen des Bildes dieser Region von Interesse von dem zweiten Speicher zu dem ersten Speicher beinhaltet;
für jede Region von Interesse aus der Subjektvielzahl von Regionen von Interesse, die sich von der anfänglichen Region von Interesse unterscheidet, das Speichern dieser Region von Interesse in dem ersten Speicher Folgendes beinhaltet:
Übertragen eines ersten Abschnitts des Bildes dieser Region von Interesse von dem zweiten Speicher zu dem ersten Speicher zu einem Zeitpunkt, zu dem der erste Speicher bereits einen zweiten Abschnitt dieser Region von Interesse enthält, als ein Ergebnis dessen, dass dieser zweite Abschnitt in einer anderen, zuvor gespeicherten Region von Interesse enthalten ist, wobei sich der erste Abschnitt des Bildes und diese Region von Interesse und der zweite Abschnitt dieser Region von Interesse kombinieren, um das Bild der Region von Interesse bereitzustellen; und
Entfernen von Daten aus dem ersten Speicher, wobei die aus dem ersten Speicher entfernten Daten durch den ersten Abschnitt des Bildes der Region von Interesse ersetzt werden;
die prozessorlose Speziallogikschaltung für jede Region von Interesse aus der Vielzahl von Regionen von Interesse die Bildqualitätsbewertung für diese Region von Interesse berechnen soll;
für mindestens eine Region von Interesse aus der Subjektvielzahl von Regionen von Interesse mindestens ein Abschnitt des zweiten Abschnitts dieser Region von Interesse aus einer Vielzahl von unterschiedlichen, zuvor gespeicherten Regionen von Interesse besteht.

10. Verfahren gemäß Anspruch 9, wobei für jede der Subjektvielzahl von Regionen von Interesse:
das Erfassen, unter Verwendung der Bildgebungsanordnung (122), des Bildes dieser Region von Interesse das Erfassen eines entsprechenden Bildes des Subjektkanals beinhaltet, wobei:
das entsprechende Bild des Subjektkanals eine Ausdehnung entlang der Breite des Subjektkanals aufweist, die größer als eine Ausdehnung der Region von Interesse entlang der Breite des Subjektkanals ist; und
das entsprechende Bild des Subjektkanals eine Ausdehnung entlang der Länge des Subjektkanals aufweist, die gleich einer Ausdehnung der Region von Interesse entlang der Länge des Subjektkanals ist;
und
das Speichern des Bildes dieser Region von Interesse in dem zweiten Speicher das Speichern des entsprechenden Bildes des Subjektkanals in dem zweiten Speicher beinhaltet.

11. Verfahren gemäß Anspruch 9, wobei:
jeder des einen oder der mehreren Kanäle (210, 310) eine erste Endregion, eine zweite Endregion und eine Zwischenregion, die sich zwischen der ersten Endregion und der zweiten Endregion erstreckt, beinhaltet;
das Verfahren Folgendes beinhaltet:
während einer ersten Periode ein Sichtfeld der Bildgebungsanordnung entlang der Länge des Subjektkanals von der ersten Endregion des Subjektkanals durch die Zwischenregion des Subjektkanals zu der zweiten Endregion des Subjektkanals zu bewegen;
Durchführen des Satzes von Kalibrierungshandlungen mit einer ersten Vielzahl von Regionen von Interesse als die Subjektvielzahl von Regionen von Interesse während der ersten Periode, wobei die erste Vielzahl von Regionen von Interesse Regionen von Interesse in der ersten Endregion des Subjektkanals sind;
während einer zweiten Periode ein Sichtfeld der Bildgebungsanordnung entlang der Länge des Subjektkanals von der zweiten Endregion des Subjektkanals durch die Zwischenregionen des Subjektkanals zu der ersten Endregion des Subjektkanals zu bewegen; und
Durchführen des Satzes von Kalibrierungshandlungen mit einer zweiten Vielzahl von Regionen als die Subjektvielzahl von Regionen von Interesse während der zweiten Periode, wobei die zweite Vielzahl von Regionen von Interesse Regionen von Interesse in der zweiten Endregion des Subjektkanals sind;
Durchführen des Satzes von Kalibrierungshandlungen mit einer dritten Vielzahl von Regionen von Interesse als die Subjektvielzahl von Regionen von Interesse, wobei die dritte Vielzahl von Regionen von Interesse Regionen von Interesse in der Zwischenregion des Subjektkanals sind; und
Bestimmen einer Nukleotidsequenz für eine Probe von biologischem Material durch Durchführen von Sequenzierung durch Synthese basierend auf Nukleotiddaten, die von der dritten Vielzahl von Regionen von Interesse erfasst werden.

12. Verfahren gemäß Anspruch 11, wobei das Verfahren Folgendes beinhaltet:
während des Durchführens des Satzes von Kalibrierungshandlungen mit der ersten Vielzahl von Regionen von Interesse als die Subjektvielzahl von Regionen von Interesse, Antreiben einer relativen Bewegung zwischen dem Merkmal der Bildgebungsanordnung (122) und der Durchflusszelle (110, 200, 300) entlang einer Höhe, wobei die Höhe senkrecht zu der Länge und Breite des Subjektkanals ist, durch einen kontinuierlichen Bewegungsbereich zwischen einem ersten Wert und einem zweiten Wert; und
während des Durchführens des Satzes von Kalibrierungshandlungen mit der dritten Vielzahl von Regionen von Interesse als die Subjektvielzahl von Regionen von Interesse, Antreiben einer relativen Bewegung zwischen dem Merkmal der Bildgebungsanordnung und der Durchflusszelle entlang der Höhe durch einen kontinuierlichen Bewegungsbereich zwischen einem dritten Wert und einem vierten Wert; und wobei der dritte Wert und der vierte Wert jeweils zwischen dem ersten Wert und dem zweiten Wert liegen.

13. Verfahren gemäß Anspruch 9, wobei:
das Merkmal der Bildgebungsanordnung (122) eine Objektivlinse (142) ist;
das Verfahren für den Subjektkanal während des Durchführens des Satzes von Kalibrierungshandlungen Folgendes beinhaltet:
Antreiben einer relativen Bewegung zwischen der Objektivlinse und der Oberfläche des Subjektkanals durch einen kontinuierlichen Bewegungsbereich entlang einer Höhe, die senkrecht zu der Länge und der Breite des Subjektkanals ist; und
Antreiben einer relativen Bewegung des Subjektkanals und des Sichtfelds der Bildgebungsanordnung entlang der Länge des Subjektkanals;
die Fokuskomponente für den Subjektkanal Bildqualitätsproxywerte für die Oberfläche dieses Kanals durch Durchführen von Handlungen erhalten soll, die das Projizieren eines Satzes von Punkten auf die Oberfläche des Kanals und das Detektieren von Reflexionen des Satzes von Punkten von der Oberfläche dieses Kanals beinhalten;
für jede Region von Interesse aus der Subjektvielzahl von Regionen von Interesse:
der eine oder die mehreren Bildqualitätsproxywerte für diese Region von Interesse einen durchschnittlichen Punkttrennungswert für diese Region von Interesse beinhalten; und
das Bestimmen des einen oder der mehreren Bildqualitätsproxywerte für diese Region von Interesse unter Verwendung der Fokuskomponente beinhaltet, dass die Fokuskomponente den Satz von Punkten auf die Oberfläche des Subjektkanals projiziert, Reflexionen des Satzes von Punkten von der Oberfläche des Subjektkanals detektiert, während das Bild dieser Region von Interesse erfasst wird; und
das Bestimmen, ob das Merkmal der Bildgebungsanordnung angepasst werden soll, das Bestimmen beinhaltet, ob relative Positionen der Objektivlinse und der Oberfläche des Subjektkanals entlang der Höhe angepasst werden sollen.

## Revendications

1. Un appareil, comprenant :
une cellule d'écoulement *(flow cell*) (300) comprenant un ou plusieurs canaux (310), où chacun des un ou plusieurs canaux a une longueur et une largeur, la longueur étant supérieure à la largeur, et comprend une surface ayant une pluralité de sites de réaction ;
un ensemble d'imagerie (122) destiné à recevoir de la lumière émise depuis un réactif positionné au niveau des sites de réaction en réponse à une lumière d'excitation ;
un composant de focalisation (162) destiné, pour chaque canal parmi les un ou plusieurs canaux, à obtenir des valeurs proxy de qualité d'image pour la surface de ce canal ; et
une circuiterie logique comprenant une première mémoire, où la circuiterie logique est configurée pour, pour un canal sujet parmi les un ou plusieurs canaux :
pour chaque région d'une pluralité sujette de régions d'intérêt, où chaque région d'intérêt est une région bidimensionnelle sur la surface du canal sujet ayant une pluralité de sites de réaction distincts les uns des autres sur la longueur du canal sujet et une pluralité de sites de réaction distincts les uns des autres sur la largeur du canal, réaliser un ensemble d'actes d'étalonnage comprenant :
la capture, à l'aide de l'ensemble d'imagerie, d'une image de cette région d'intérêt ;
le stockage de l'image de cette région d'intérêt dans une première mémoire ;
la détermination d'une ou de plusieurs valeurs proxy de qualité d'image pour cette région d'intérêt à l'aide du composant de focalisation ; et
le calcul d'un score de qualité d'image pour cette région d'intérêt ;
générer une courbe d'étalonnage reliant des scores de qualité d'image pour les régions d'intérêt à des valeurs proxy de qualité d'image pour les régions d'intérêt ; et
pendant l'entraînement d'un mouvement relatif du canal sujet et d'un champ de vision de l'ensemble d'imagerie sur la longueur du canal sujet par l'intermédiaire d'un ou de plusieurs actionneurs d'un étage d'échantillon sur lequel la cellule d'écoulement est montée ou par l'intermédiaire d'un ou de plusieurs actionneurs de l'ensemble d'imagerie, réaliser un ensemble d'actes d'appel de base comprenant :
l'obtention de données nucléotidiques sur la base de l'utilisation de l'ensemble d'imagerie pour détecter de la lumière émise depuis des réactifs positionnés au niveau de sites de réaction sur la surface du canal sujet ;
l'obtention d'une ou de plusieurs valeurs proxy de qualité d'image à l'aide du composant de focalisation pendant l'obtention de données nucléotidiques ; et
sur la base de la courbe d'étalonnage et des une ou plusieurs valeurs proxy de qualité d'image obtenues pendant l'obtention de données nucléotidiques, la détermination s'il convient de régler un élément de l'ensemble d'imagerie
où :
la circuiterie logique comprend :
un processeur d'emploi général programmé ; et
un circuit logique d'emploi spécial sans processeur ;
la première mémoire est une mémoire locale résidant sur le circuit logique d'emploi spécial sans processeur ;
l'appareil comprend une deuxième mémoire connectée de manière opérationnelle au processeur d'emploi général programmé ;
pour chaque région d'intérêt parmi la pluralité sujette de régions d'intérêt, l'ensemble d'actes d'étalonnage comprend, avant le stockage de l'image de cette région d'intérêt dans la première mémoire, le stockage de l'image de cette région d'intérêt dans la deuxième mémoire ;
pour une région d'intérêt initiale parmi la pluralité sujette de régions d'intérêt, le stockage de l'image de cette région d'intérêt dans la première mémoire comprend le transfert de l'image de cette région d'intérêt de la deuxième mémoire à la première mémoire ;
pour chaque région d'intérêt parmi la pluralité sujette de régions d'intérêt autre que la région d'intérêt initiale, le stockage de cette région d'intérêt dans la première mémoire comprend :
le transfert d'une première partie de l'image de cette région d'intérêt de la deuxième mémoire à la première mémoire à un moment auquel la première mémoire contient déjà une deuxième partie de cette région d'intérêt du fait que cette deuxième partie est constituée d'une région d'intérêt différente,
précédemment stockée, où la première partie de l'image et de cette région d'intérêt et la deuxième partie de cette région d'intérêt se combinent pour fournir l'image de la région d'intérêt ; et
le retrait de données de la première mémoire, où les données retirées de la première mémoire sont remplacées par la première partie de l'image de la région d'intérêt ;
le circuit logique d'emploi spécial sans processeur est configuré pour, pour chaque région d'intérêt parmi la pluralité de régions d'intérêt, calculer le score de qualité d'image pour cette région d'intérêt.

2. L'appareil de la revendication 1, où pour chaque région d'intérêt parmi la pluralité sujette de régions d'intérêt, cette région d'intérêt est en chevauchement avec au moins une autre région d'intérêt de la pluralité sujette de régions d'intérêt sur la longueur du canal sujet.

3. L'appareil de la revendication 1, où, pour au moins une région d'intérêt parmi la pluralité sujette de régions d'intérêt, au moins une partie de la deuxième partie de cette région d'intérêt est constituée d'une pluralité de régions d'intérêt différentes, précédemment stockées.

4. L'appareil de la revendication 1, où, pour chaque région de la pluralité sujette de régions d'intérêt :
la capture, à l'aide de l'ensemble d'imagerie (122), de l'image de cette région d'intérêt comprend la capture d'une image correspondante du canal sujet, où :
l'image correspondante du canal sujet a une étendue sur la largeur du canal sujet supérieure à une étendue de la région d'intérêt sur la largeur du canal sujet ; et
l'image correspondante du canal sujet a une étendue sur la longueur du canal sujet égale à une étendue de la région d'intérêt sur la longueur du canal sujet ;
et
le stockage de l'image de cette région d'intérêt dans la deuxième mémoire comprend le stockage de l'image correspondante du canal sujet dans la deuxième mémoire.

5. L'appareil de la revendication 1, où :
chacun des un ou plusieurs canaux (210, 310) comprend une première région d'extrémité, une deuxième région d'extrémité, et une région intermédiaire s'étendant entre la première région d'extrémité et la deuxième région d'extrémité ;
la circuiterie logique est configurée pour :
pendant une première période, déplacer un champ de vision de l'ensemble d'imagerie sur la longueur du canal sujet de la première région d'extrémité du canal sujet, à travers la région intermédiaire du canal sujet, à la deuxième région d'extrémité du canal sujet ;
réaliser l'ensemble d'actes d'étalonnage avec une première pluralité de régions d'intérêt comme pluralité sujette de régions d'intérêt pendant la première période, où la première pluralité de régions d'intérêt sont des régions d'intérêt dans la première région d'extrémité du canal sujet ;
pendant une deuxième période, déplacer un champ de vision de l'ensemble d'imagerie sur la longueur du canal sujet de la deuxième région d'extrémité du canal sujet, à travers les régions intermédiaires du canal sujet, à la première région d'extrémité du canal sujet ; et
réaliser l'ensemble d'actes d'étalonnage avec une deuxième pluralité de régions comme pluralité sujette de régions d'intérêt pendant la deuxième période, où la deuxième pluralité de régions d'intérêt sont des régions d'intérêt dans la deuxième région d'extrémité du canal sujet.

6. L'appareil de la revendication 5, où la circuiterie logique est configurée pour réaliser l'ensemble d'actes d'étalonnage avec une troisième pluralité de régions d'intérêt comme pluralité sujette de régions d'intérêt, où la troisième pluralité de régions d'intérêt sont des régions d'intérêt dans la région intermédiaire du canal sujet.

7. L'appareil de la revendication 6, où la circuiterie logique est configurée pour :
pendant la réalisation de l'ensemble d'actes d'étalonnage avec la première pluralité de régions d'intérêt comme pluralité sujette de régions d'intérêt, entraîner un déplacement relatif entre l'élément de l'ensemble d'imagerie (122) et la cellule d'écoulement (110) sur une hauteur, où la hauteur est perpendiculaire à la longueur et à la largeur du canal sujet, sur une plage continue de mouvement entre une première valeur et une deuxième valeur ; et
pendant la réalisation de l'ensemble d'actes d'étalonnage avec la troisième pluralité de régions d'intérêt comme pluralité sujette de régions d'intérêt, entraîner un déplacement relatif entre l'élément de l'ensemble d'imagerie (122) et la cellule d'écoulement (110) sur la hauteur sur une plage continue de mouvement entre une troisième valeur et une quatrième valeur, où la troisième valeur et la quatrième valeur sont chacune entre la première valeur et la deuxième valeur.

8. L'appareil de la revendication 1, où :
l'élément de l'ensemble d'imagerie (122) est une lentille d'objectif (142) ;
la circuiterie logique est configurée pour, pour le canal sujet parmi les un ou plusieurs canaux (210, 310), pendant la réalisation de l'ensemble d'actes d'étalonnage :
entraîner un déplacement relatif entre la lentille d'objectif (142) et la surface du canal sujet sur une plage continue de mouvement sur une hauteur qui est perpendiculaire à la longueur et à la largeur du canal sujet ; et
entraîner un mouvement relatif du canal sujet et du champ de vision de l'ensemble d'imagerie (122) sur la longueur du canal sujet ;
le composant de focalisation (162) est configuré pour, pour chaque canal parmi les un ou plusieurs canaux, obtenir des valeurs proxy de qualité d'image pour la surface de ce canal en réalisant des actes comprenant la projection d'un ensemble de points sur la surface du canal, et la détection de réflexions de l'ensemble de points depuis la surface de ce canal ;
pour chaque région d'intérêt parmi la pluralité sujette de régions d'intérêt :
les une ou plusieurs valeurs proxy de qualité d'image pour cette région d'intérêt comprennent une valeur de séparation entre points moyenne pour cette région d'intérêt ; et
la détermination des une ou plusieurs valeurs proxy de qualité d'image pour cette région d'intérêt à l'aide du composant de focalisation comprend, par le composant de focalisation, la projection de l'ensemble de points sur, la détection de réflexions de l'ensemble de points depuis, la surface du canal sujet pendant la capture de l'image de cette région d'intérêt ; et
la détermination s'il convient de régler l'élément de l'ensemble d'imagerie comprend la détermination s'il convient de régler des positions relatives de la lentille d'objectif et de la surface du canal sujet sur la hauteur.

9. Un procédé comprenant :
pour chaque région d'une pluralité sujette de régions d'intérêt, la réalisation d'un ensemble d'actes d'étalonnage, où chaque région d'intérêt est une région bidimensionnelle sur une surface d'un canal sujet d'une cellule d'écoulement (110, 200, 300),
le canal sujet ayant une pluralité de sites de réaction distincts les uns des autres sur la longueur du canal sujet et une pluralité de sites de réaction distincts les uns des autres sur la largeur du canal, la réalisation d'un ensemble d'actes d'étalonnage comprenant :
la capture, à l'aide d'un ensemble d'imagerie (122), d'une image de cette région d'intérêt ;
le stockage de l'image de cette région d'intérêt dans une première mémoire ;
la détermination d'une ou de plusieurs valeurs proxy de qualité d'image pour cette région d'intérêt à l'aide d'un composant de focalisation (162) d'un système pour l'analyse de matériaux chimiques ou biologiques ;
le calcul d'un score de qualité d'image pour cette région d'intérêt ;
la génération d'une courbe d'étalonnage reliant des scores de qualité d'image pour les régions d'intérêt à des valeurs proxy de qualité d'image pour les régions d'intérêt ;
pendant l'entraînement d'un mouvement relatif du canal sujet et d'un champ de vision de l'ensemble d'imagerie sur la longueur du canal sujet par l'intermédiaire d'un ou de plusieurs actionneurs d'un étage d'échantillon sur lequel la cellule d'écoulement est montée ou par l'intermédiaire d'un ou de plusieurs actionneurs de l'ensemble d'imagerie, la réalisation d'un ensemble d'actes d'appel de base comprenant :
l'obtention de données nucléotidiques sur la base de l'utilisation de l'ensemble d'imagerie pour détecter de la lumière émise depuis des réactifs positionnés au niveau de sites de réaction sur la surface du canal sujet ;
l'obtention d'une ou de plusieurs valeurs proxy de qualité d'image à l'aide du composant de focalisation pendant l'obtention de données nucléotidiques ; et
sur la base de la courbe d'étalonnage et des une ou plusieurs valeurs proxy de qualité d'image obtenues pendant l'obtention de données nucléotidiques, la détermination s'il convient de régler un élément de l'ensemble d'imagerie ;
où :
la première mémoire est une mémoire locale résidant sur un circuit logique d'emploi spécial sans processeur ;
pour chaque région d'intérêt parmi la pluralité sujette de régions d'intérêt, l'ensemble d'actes d'étalonnage comprend, avant le stockage de l'image de cette région d'intérêt dans la première mémoire, le stockage de l'image de cette région d'intérêt dans une deuxième mémoire, où la deuxième mémoire est connectée de manière opérationnelle à un processeur d'emploi général ;
pour une région d'intérêt initiale parmi la pluralité sujette de régions d'intérêt, le stockage de l'image de cette région d'intérêt dans la première mémoire comprend le transfert de l'image de cette région d'intérêt de la deuxième mémoire à la première mémoire ;
pour chaque région d'intérêt parmi la pluralité sujette de régions d'intérêt autre que la région d'intérêt initiale, le stockage de cette région d'intérêt dans la première mémoire comprend :
le transfert d'une première partie de l'image de cette région d'intérêt de la deuxième mémoire à la première mémoire à un moment auquel la première mémoire contient déjà une deuxième partie de cette région d'intérêt du fait que cette deuxième partie est constituée d'une région d'intérêt différente, précédemment stockée, où la première partie de l'image et de cette région d'intérêt et la deuxième partie de cette région d'intérêt se combinent pour fournir l'image de la région d'intérêt ; et
le retrait de données de la première mémoire, où les données retirées de la première mémoire sont remplacées par la première partie de l'image de la région d'intérêt ;
le circuit logique d'emploi spécial sans processeur est destiné, pour chaque région d'intérêt parmi la pluralité de régions d'intérêt, à calculer le score de qualité d'image pour cette région d'intérêt ;
pour au moins une région d'intérêt parmi la pluralité sujette de régions d'intérêt, au moins une partie de la deuxième partie de cette région d'intérêt est constituée d'une pluralité de régions d'intérêt différentes, précédemment stockées.

10. Le procédé de la revendication 9, où, pour chaque région de la pluralité sujette de régions d'intérêt :
la capture, à l'aide de l'ensemble d'imagerie (122), de l'image de cette région d'intérêt comprend la capture d'une image correspondante du canal sujet, où :
l'image correspondante du canal sujet a une étendue sur la largeur du canal sujet supérieure à une étendue de la région d'intérêt sur la largeur du canal sujet ; et
l'image correspondante du canal sujet a une étendue sur la longueur du canal
sujet égale à une étendue de la région d'intérêt sur la longueur du canal sujet ;
et
le stockage de l'image de cette région d'intérêt dans la deuxième mémoire comprend le stockage de l'image correspondante du canal sujet dans la deuxième mémoire.

11. Le procédé de la revendication 9, où :
chacun des un ou plusieurs canaux (210, 310) comprend une première région d'extrémité, une deuxième région d'extrémité, et une région intermédiaire s'étendant entre la première région d'extrémité et la deuxième région d'extrémité ;
le procédé comprend :
pendant une première période, le déplacement d'un champ de vision de l'ensemble d'imagerie sur la longueur du canal sujet de la première région d'extrémité du canal sujet, à travers la région intermédiaire du canal sujet, à la deuxième région d'extrémité du canal sujet ;
la réalisation de l'ensemble d'actes d'étalonnage avec une première pluralité de régions d'intérêt comme pluralité sujette de régions d'intérêt pendant la première période, où la première pluralité de régions d'intérêt sont des régions d'intérêt dans la première région d'extrémité du canal sujet ;
pendant une deuxième période, le déplacement d'un champ de vision de l'ensemble d'imagerie sur la longueur du canal sujet de la deuxième région d'extrémité du canal sujet, à travers les régions intermédiaires du canal sujet, à la première région d'extrémité du canal sujet ; et
la réalisation de l'ensemble d'actes d'étalonnage avec une deuxième pluralité de régions comme pluralité sujette de régions d'intérêt pendant la deuxième période, où la deuxième pluralité de régions d'intérêt sont des régions d'intérêt dans la deuxième région d'extrémité du canal sujet ;
la réalisation de l'ensemble d'actes d'étalonnage avec une troisième pluralité de régions d'intérêt comme pluralité sujette de régions d'intérêt, où la troisième pluralité de régions d'intérêt sont des régions d'intérêt dans la région intermédiaire du canal sujet ; et
la détermination d'une séquence nucléotidique pour un échantillon de matériau biologique en réalisant un séquençage par synthèse sur la base de données nucléotidiques capturées à partir de la troisième pluralité de régions d'intérêt.

12. Le procédé de la revendication 11, où le procédé comprend :
pendant la réalisation de l'ensemble d'actes d'étalonnage avec la première pluralité de régions d'intérêt comme pluralité sujette de régions d'intérêt, l'entraînement d'un déplacement relatif entre l'élément de l'ensemble d'imagerie (122) et la cellule d'écoulement (110, 200, 300) sur une hauteur, où la hauteur est perpendiculaire à la longueur et à la largeur du canal sujet, sur une plage continue de mouvement entre une première valeur et une deuxième valeur ; et
pendant la réalisation de l'ensemble d'actes d'étalonnage avec la troisième pluralité de régions d'intérêt comme pluralité sujette de régions d'intérêt, l'entraînement d'un déplacement relatif entre l'élément de l'ensemble d'imagerie et la cellule d'écoulement sur la hauteur sur une plage continue de mouvement entre une troisième valeur et une quatrième valeur, où la troisième valeur et la quatrième valeur sont chacune entre la première valeur et la deuxième valeur.

13. Le procédé de la revendication 9, où :
l'élément de l'ensemble d'imagerie (122) est une lentille d'objectif (142) ;
le procédé comprend pour le canal sujet, pendant la réalisation de l'ensemble d'actes d'étalonnage :
l'entraînement d'un déplacement relatif entre la lentille d'objectif et la surface du canal sujet sur une plage continue de mouvement sur une hauteur qui est perpendiculaire à la longueur et à la largeur du canal sujet ; et
l'entraînement d'un mouvement relatif du canal sujet et du champ de vision de l'ensemble d'imagerie sur la longueur du canal sujet ;
le composant de focalisation est destiné, pour le canal sujet, à obtenir des valeurs proxy de qualité d'image pour la surface de ce canal en réalisant des actes comprenant la projection d'un ensemble de points sur la surface du canal, et la détection de réflexions de l'ensemble de points depuis la surface de ce canal ;
pour chaque région d'intérêt parmi la pluralité sujette de régions d'intérêt :
les une ou plusieurs valeurs proxy de qualité d'image pour cette région d'intérêt comprennent une valeur de séparation entre points moyenne pour cette région d'intérêt ; et
la détermination des une ou plusieurs valeurs proxy de qualité d'image pour cette région d'intérêt à l'aide du composant de focalisation comprend, par le composant de focalisation, la projection de l'ensemble de points sur, la détection de réflexions de l'ensemble de points depuis, la surface du canal sujet pendant la capture de l'image de cette région d'intérêt ; et
la détermination s'il convient de régler l'élément de l'ensemble d'imagerie comprend la détermination s'il convient de régler des positions relatives de la lentille d'objectif et de la surface du canal sujet sur la hauteur.
